# EUROPÄISCHE PATENTSCHRIFT

(11) **EP 3 099 787 B1**
(45) Veröffentlichungstag und Bekanntmachung des Hinweises auf die Patenterteilung: **19.12.2018**
(21) Anmeldenummer: 15704954.5
(22) Anmeldetag: 24.01.2015
(51) Int. Cl.: C12N 5/0797

(54) **BEFELDUNG NEURONALER ZELLKULTUREN**
IRRADIATION OF NEURAL CELL CULTURES
APPLICATION D'UN CHAMP À DES STRUCTURES CELLULAIRES NEURONALES

(30) Priorität: 31.01.2014 DE 102014001433
(43) Veröffentlichungstag der Anmeldung: 07.12.2016
(73) Patentinhaber: Mega-Wave-GmbH, 86825 Bad Wörishofen (DE)
(72) Erfinder: PRIES, Wolfgang Habil, 86825 Bad Wörishofen (DE)
(74) Vertreter: Liebl, Thomas
(86) Internationale Anmeldenummer: PCT/EP2015/000137
(87) Internationale Veröffentlichungsnummer: WO 2015/113751

(56) Entgegenhaltungen:
- EP-A1- 2 153 869
- US-A1- 2013 202 565
- MARVI A. MATOS ET AL: "Alternating current electric field effects on neural stem cell viability and differentiation", BIOTECHNOLOGY PROGRESS, Bd. 26, Nr. 3, 4. Mai 2010 (2010-05-04), Seiten 664-670, XP055174701, ISSN: 8756-7938, DOI: 10.1002/btpr.389
- NORITAKA NAKAMICHI ET AL: "Possible promotion of neuronal differentiation in fetal rat brain neural progenitor cells after sustained exposure to static magnetism", JOURNAL OF NEUROSCIENCE RESEARCH, Bd. 87, Nr. 11, 15. August 2009 (2009-08-15), Seiten 2406-2417, XP055174736, ISSN: 0360-4012, DOI: 10.1002/jnr.22087
- QINLONG MA ET AL: "Extremely Low-Frequency Electromagnetic Fields Affect Transcript Levels of Neuronal Differentiation-Related Genes in Embryonic Neural Stem Cells", PLOS ONE, Bd. 9, Nr. 3, 3. März 2014 (2014-03-03), Seite e90041, XP055174752, DOI: 10.1371/journal.pone.0090041
- KOTANI S ET AL: "Donepezil, an acetylcholinesterase inhibitor, enhances adult hippocampal neurogenesis", CHEMICO-BIOLOGICAL INTERACTIONS, ELSEVIER SCIENCE IRLAND, IR, Bd. 175, Nr. 1-3, 25. September 2008 (2008-09-25), Seiten 227-230, XP024530536, ISSN: 0009-2797, DOI: 10.1016/J.CBI.2008.04.004 [gefunden am 2008-04-12]

## Beschreibung

### Gebiet der Erfindung

Die Erfindung betrifft ein *in vitro* Verfahren zur Förderung der Entwicklung von Neuronen, umfassend die Schritte Kultivieren der Neurone aus neuronalen Vorläuferzellen, und Befelden der neuronalen Vorläuferzellen und/oder der Neurone während des Kultivierens mit einer Hochfrequenzschwingung, die eine Frequenz von 100 bis 200 MHz aufweist.

### Hintergrund der Erfindung

Nachdem die Entwicklung des zentralen Nervensystems abgeschlossen ist, werden nur noch wenige Neuronen gebildet, so dass im adulten Organismus kaum Neurone nachgebildet und in die bestehenden neuronalen Netzwerke eingegliedert werden. Ebenso ist die Regeneration oder Neubildung von Nervenzellen im peripheren Nervensystem, beispielsweise nach einer Verletzung, nur eingeschränkt möglich. Deshalb wird nach Wegen gesucht, die Bildung von Neuronen im adulten Gewebe zu fördern. Alternativ hierzu wird versucht neuronale Zellen *in vitro* zu züchten, welche dann zur Transplantation verwendet werden sollen. Hierzu werden neuronale Vorläuferzellen in hierfür entwickelten Kulturgefäßen und Nährmedien kultiviert. Um Vorläuferzellen zur Bildung von funktionellen Neuronen zu veranlassen, ist häufig die Zugabe von Wachstumsfaktoren und Signalmolekülen notwendig, da ansonsten überwiegend Glia Zellen, das heißt Astrozyten und Oligodendrozyten, gebildet werden oder aber die gebildeten Neurone nicht vollständig ausdifferenzieren. Die Zugabe von Wachstumsfaktoren und Signalmolekülen führt jedoch häufig zu einer sehr artifiziellen Umgebung, die nur bedingt geeignet ist, Zellen hervorzubringen, die den im Gewebe enthaltenen Zellen tatsächlichen entsprechen.

Es besteht daher ein Bedarf, die Bildung neuronaler Zellen in einer möglichst physiologischen Umgebung zu fördern.

Aus der US 2013/0202565 A1 ist bereits ein Verfahren bekannt, bei dem mesenchymale Stammzellen einem elektromagnetischen Feld ausgesetzt werden, wobei bei einer Frequenz von 50 oder 100HZ die Differenzierung der Zellen in neuronale Zellen erhöht wird.

In Marvi A. Matos et al.: "Alternating current electric field effects on neural stem cell viability and differentiation", BIOTECHNOLOGY PROGRESS, Band 26, Nr. 3, 4. Mai 2010, Seiten 664 bis 670, wird ein Verfahren beschreiben, bei dem der Effekt von einem elektrischen Feld auf die Differenzierung und Lebensdauer von neuronalen Stammzellen analysiert wird, wobei bei einer Stärke von 1Hz die Lebensdauer der Zellen erhöht und die Differenzierung in Astrozyten gefördert wird.

In Noritaka Nakamichi et al.: "Possible promotion of neuronal differentiation in fetal rat brain neural progenitor cells after sustained exposure to static magnetism", Journal of Neuroscience Research, Band 87, Nr. 11, 15. August 2009, Seiten 2406 bis 2417, werden neuronale Stammzellen aus embryonalen Ratten Neokortex isoliert und unter Einfluss von einem 100mT starken Magnetfeld kultiviert, wobei das Magnetfeld bewirkt, dass sich die Zellen weniger proliferieren und eher differenzieren.

### Zusammenfassung der Erfindung

Die Erfindung betrifft ein *in vitro* Verfahren zur Förderung der Entwicklung von Neuronen, umfassend die Schritte Kultivieren der Neurone aus neuronalen Vorläuferzellen, und Befelden der neuronalen Vorläuferzellen und/oder der Neurone während des Kultivierens mit einer Hochfrequenzschwingung, die eine Frequenz von 100 bis 200 MHz aufweist, bei dem die Hochfrequenzschwingung durch eine Niederfrequenzschwingung, die eine Frequenz von 5 bis 20 Hz aufweist, moduliert wird.

### Kurzbeschreibung der Figuren

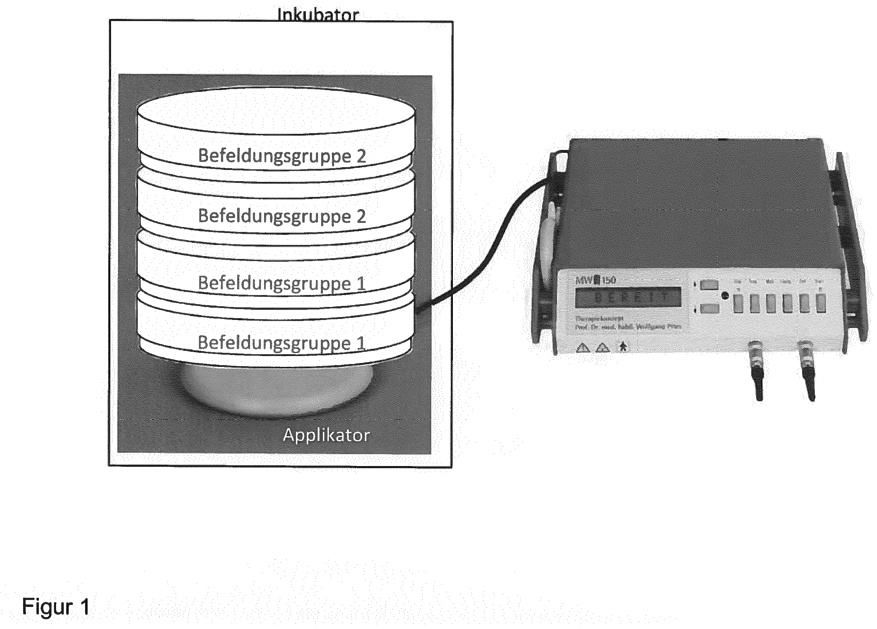

**Figur 1** zeigt eine Abbildung des Versuchsaufbaus. Links: Stapel der Petrischalen mit Mikroelektrodenarrays (MEA); Rechts: Befeldungsgerät (Mega Wave GmbH, Deutschland).

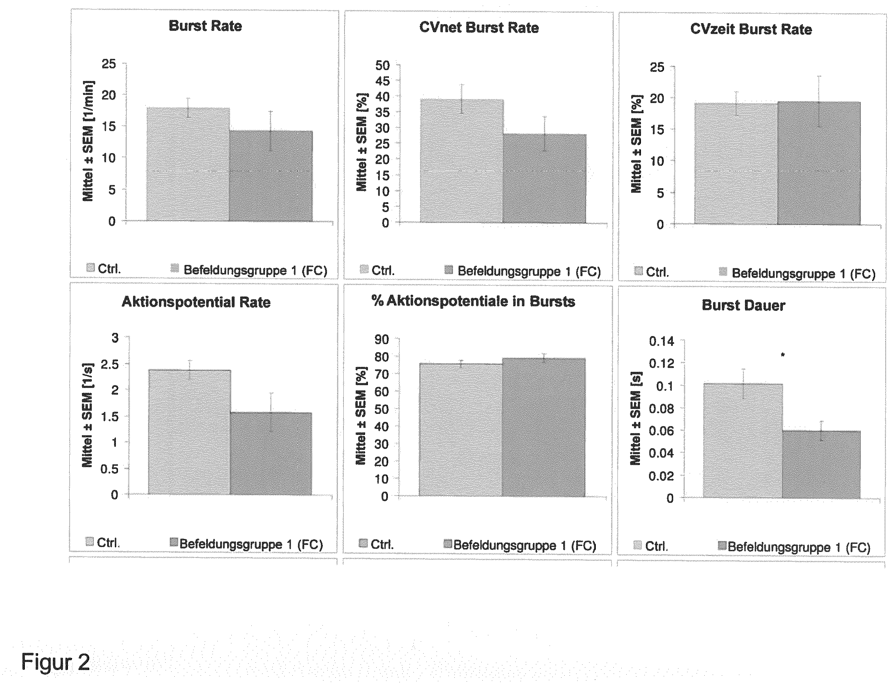

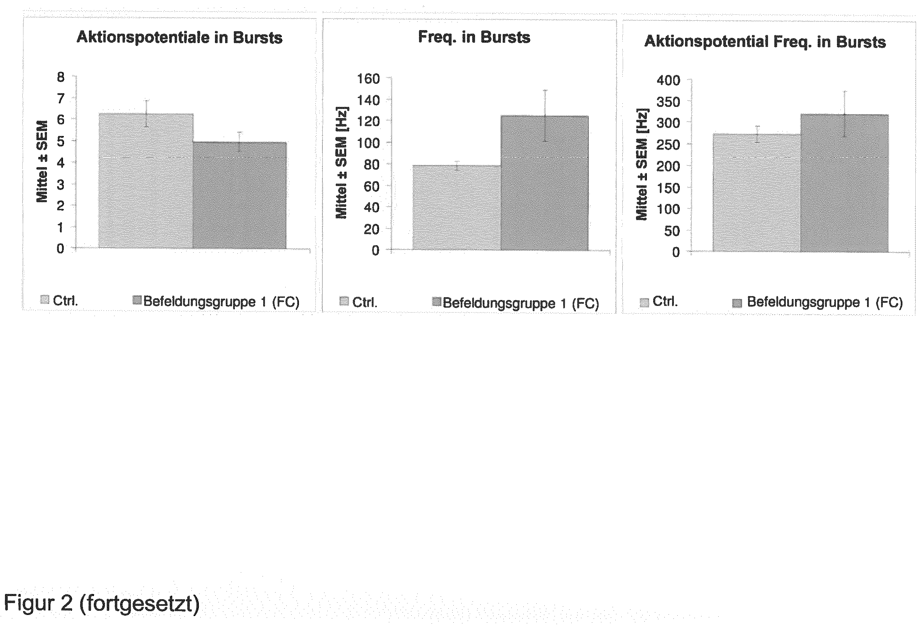

**Figur 2** zeigt die durch effektive chronische 16 Hz Befeldung induzierten Effekte (Experiment 1, Befeldungsgruppe 1) auf die kortikale Netzwerkaktivität *in vitro* im Vergleich zur unbefeldeten Kontrollaktivität. Dargestellt sind 9 Parameter in 4 Kategorien (allgemeine Aktivität, Burst Struktur, Oszillation, Synchronität). (Mittelwert ± Standardfehler (SEM), Kontrolle (Ctrl): n=13, Befeldungsgruppe 1: n=7. ungepaarter Student'scher t - Test relativ zur Kontrolle: * p ≤ 0.05; ** p ≤ 0.01; *** p ≤ 0.001)

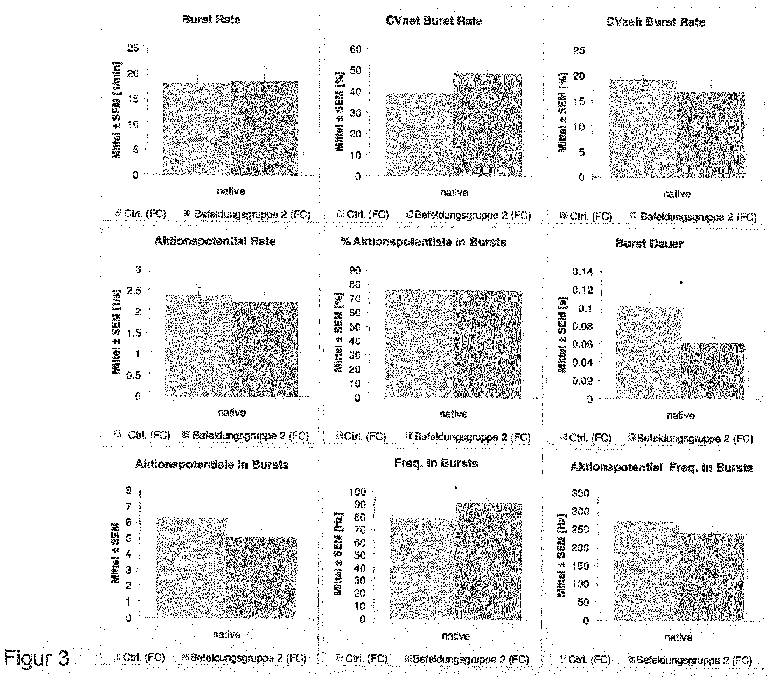

**Figur 3** zeigt die durch schwächere chronische 16 Hz Befeldung induzierten Effekte (Experiment 1, Befeldungsgruppe 2) auf die kortikale Netzwerkaktivität *in vitro* im Vergleich zur unbefeldeten Kontrollaktivität. Dargestellt sind 9 Parameter in 4 Kategorien (allgemeine Aktivität, Burst Struktur, Oszillation, Synchronität). (Mittelwert ± Standardfehler (SEM), Kontrolle (Ctrl): n=13, Befeldungsgruppe 2: n=4. ungepaarter Student'scher t - Test relativ zur Kontrolle: * p ≤ 0.05; ** p ≤ 0.01; *** p ≤ 0.001)

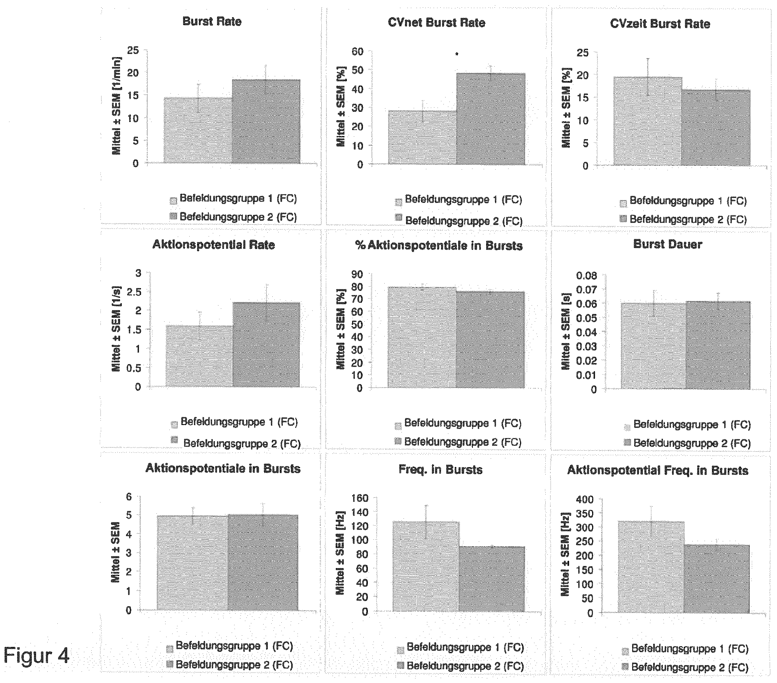

**Figur 4** zeigt den Vergleich der durch effektive und schwächere chronische 16 Hz Befeldung induzierten Effekte (Experiment 1, Befeldungsgruppe 1 und 2) auf die kortikale Netzwerkaktivität *in vitro.* Dargestellt sind 9 Parameter in 4 Kategorien (allgemeine Aktivität, Burst Struktur, Oszillation, Synchronität). (Mittelwert ± Standardfehler (SEM), Befeldungsgruppe 1: n=7, Befeldungsgruppe 2 n=4. ungepaarter Student'scher t - Test relativ zur Kontrolle: * p ≤ 0.05; ** p ≤ 0.01; *** p ≤ 0.001)

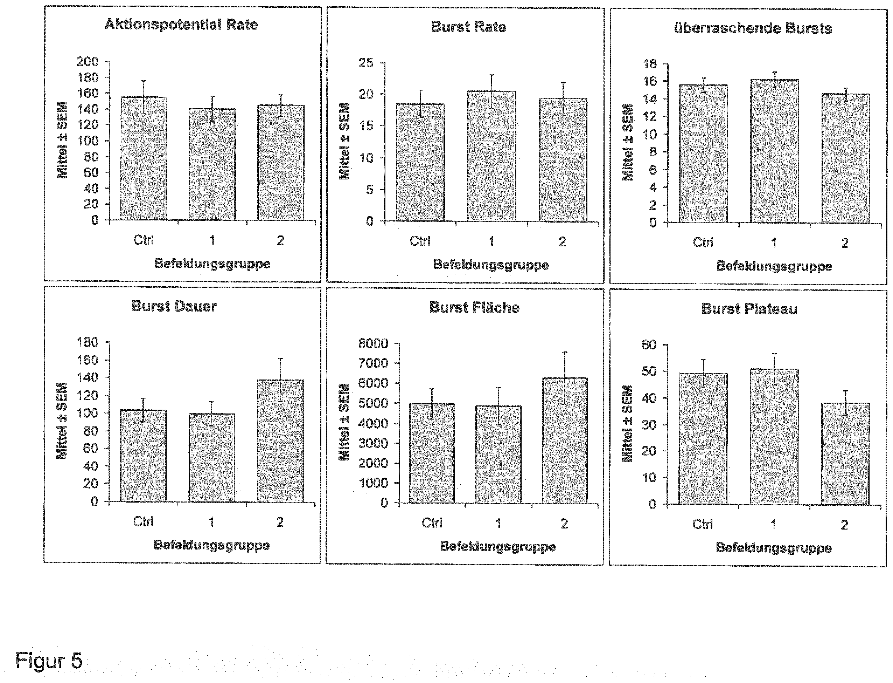

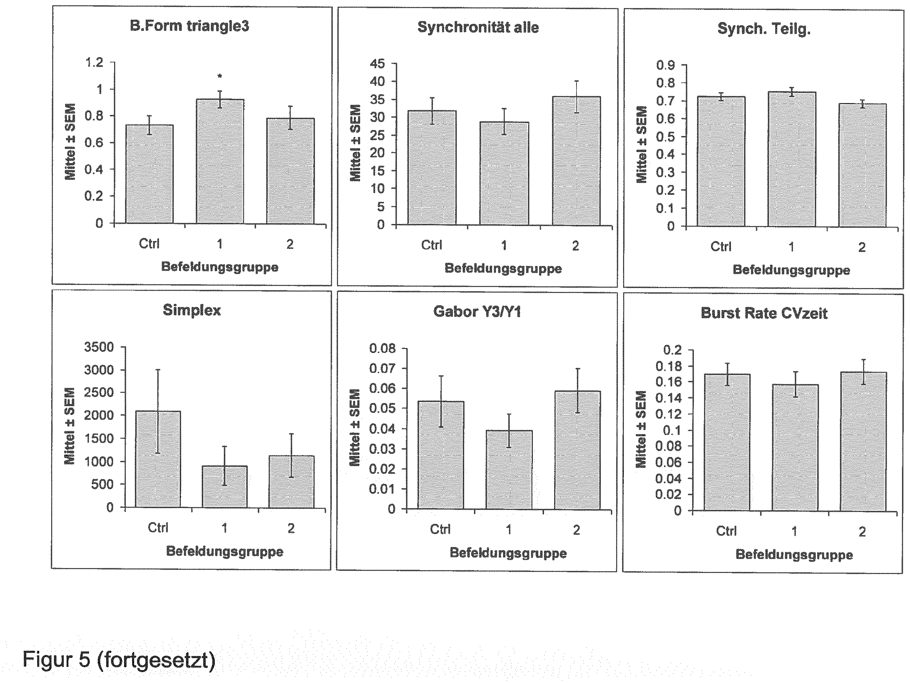

**Figur 5** zeigt die durch effektive chronische 16 Hz Befeldung induzierten Effekte (Experiment 2, Befeldungsgruppe 1 und 2) auf die kortikale Netzwerkaktivität *in vitro* im Vergleich zur unbefeldeten Kontrollaktivität. Dargestellt sind 12 Parameter in 4 Kategorien (allgemeine Aktivität, Burst Struktur, Oszillation, Synchronität). (Mittelwert ± Standardfehler (SEM), Kontrolle (Ctrl): n=18, Befeldungsgruppe 1: n=17, Befeldungsgruppe 2: n=15. ungepaarter Student'scher t - Test relativ zur Kontrolle: * p ≤ 0.05; ** p ≤ 0.01; *** p ≤ 0.001)

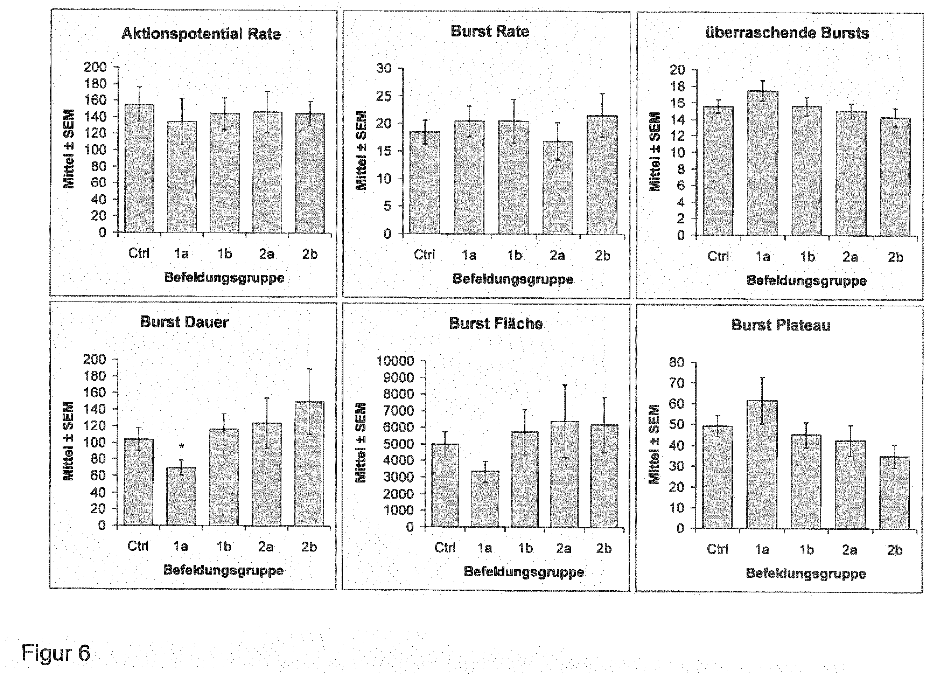

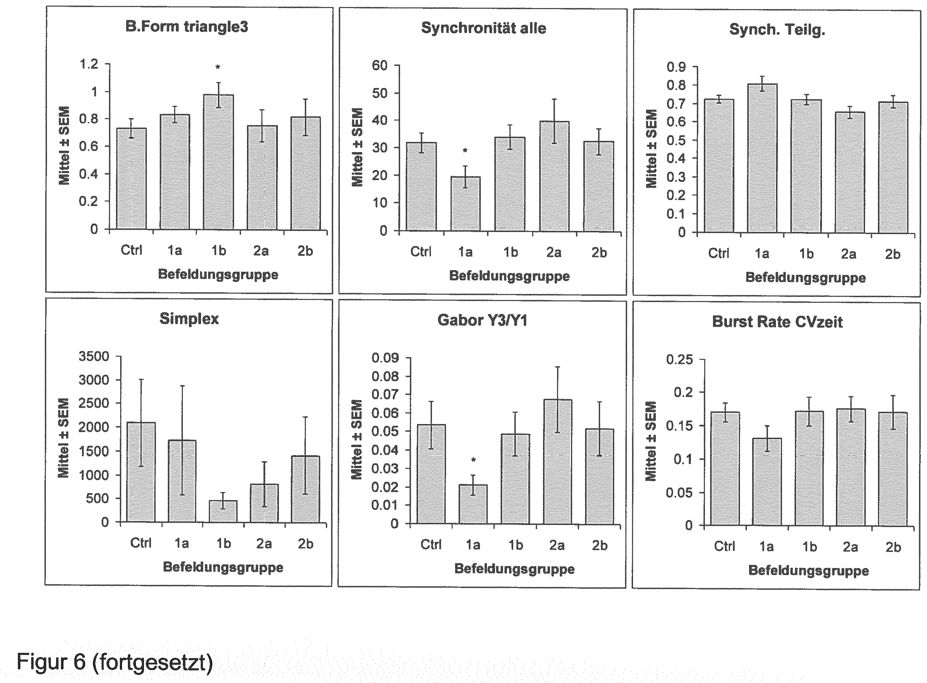

**Figur 6** zeigt die durch effektive chronische 16 Hz Befeldung induzierten Effekte (Experiment 2, Befeldungsgruppe 1a,b und 2a,b) auf die kortikale Netzwerkaktivität *in vitro* im Vergleich zur unbefeldeten Kontrollaktivität. Dargestellt sind 12 Parameter in 4 Kategorien (allgemeine Aktivität, Burst Struktur, Oszillation, Synchronität). (Mittelwert ± Standardfehler (SEM), Kontrolle (Ctrl): n=18, Befeldungsgruppe 1a: n=6, Befeldungsgruppe 1b: n=11, Befeldungsgruppe 2a: n=7, Befeldungsgruppe 2b: n=8. ungepaarter Student'scher t - Test relativ zur Kontrolle: * p ≤ 0.05; ** p ≤ 0.01; *** p ≤ 0.001)

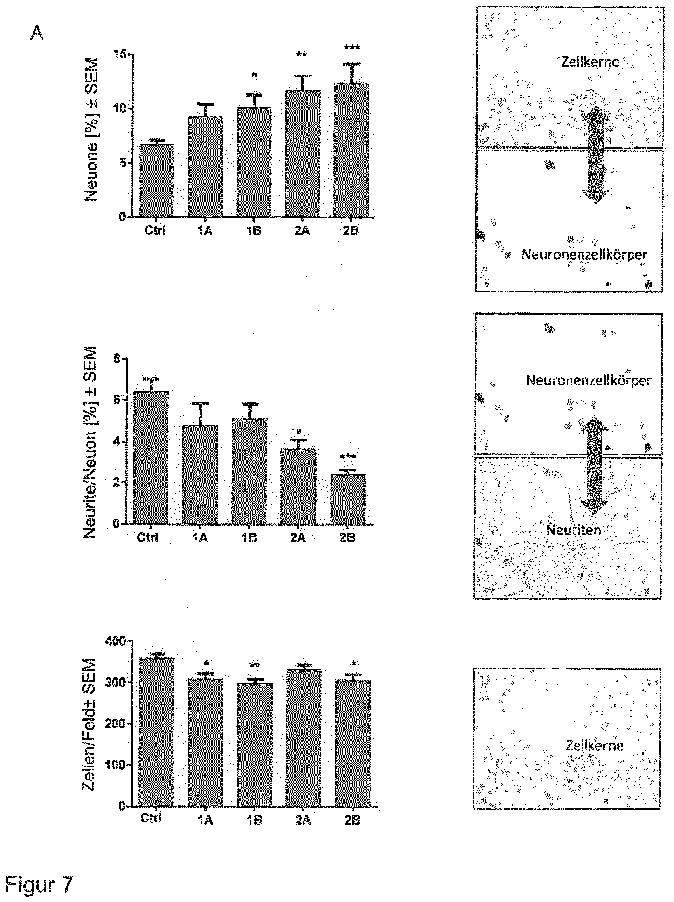

**Figur 7** zeigt den Anteil an Neuronen an der Gesamtzellzahl (A), den Anteil an Neuriten je Neuron (global je Bild) (B), und die absolute Gesamtzellzahl je Bild (C). (Experiment 2, One-way ANOVA + Dunnett's post hoc Test gegen Kontolle (Ctrl): * p<0.05, ** p<0.01, *** p<0.001)

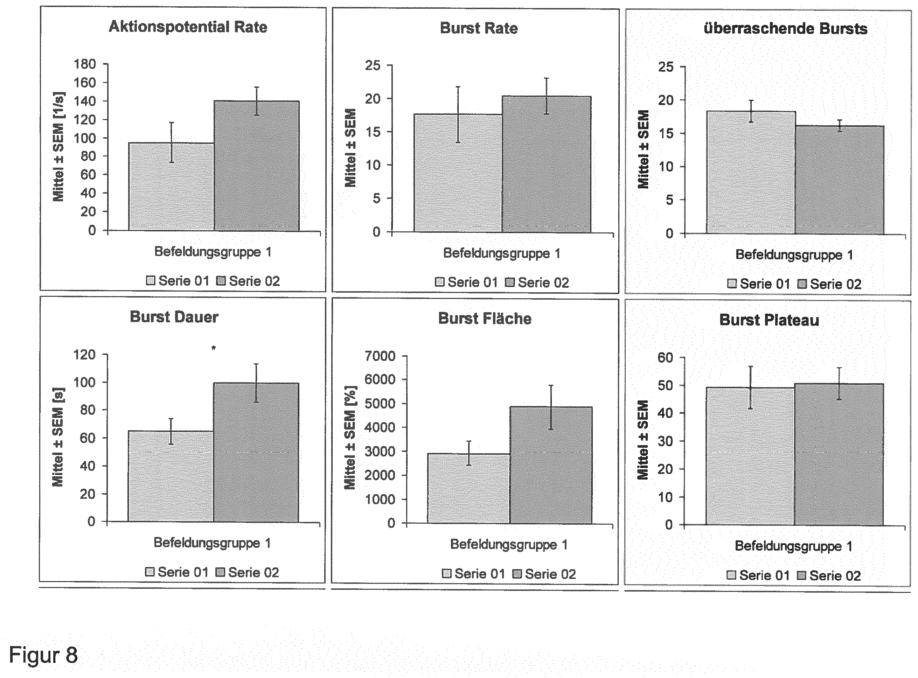

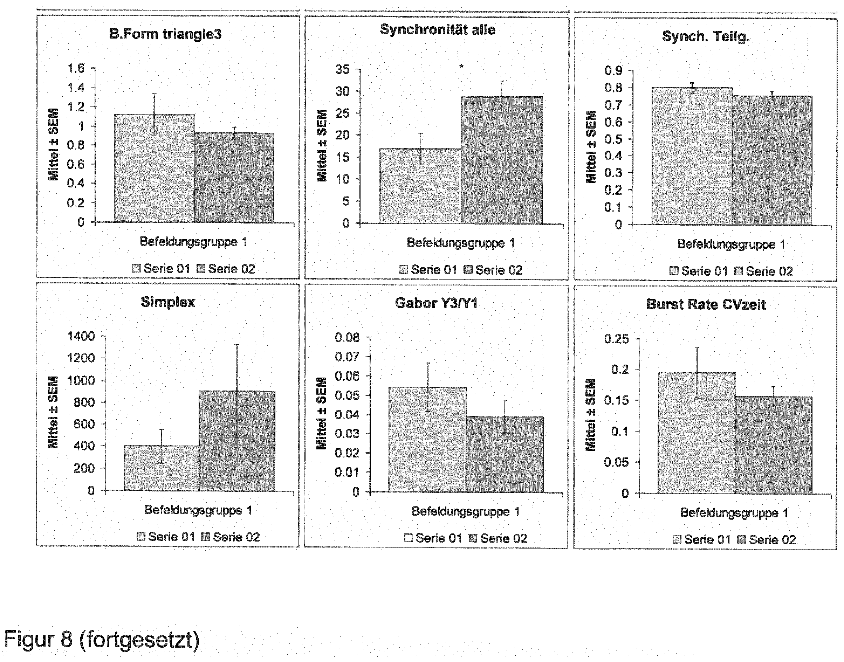

**Figur 8** zeigt die durch effektive chronische 16 Hz Befeldung induzierten Effekte (Experiment 2, Befeldungsgruppe 1, Serie 01= Leistungsstufe 6, Serie 02 = Leistungsstufe 3) auf die kortikale Netzwerkaktivität *in vitro*. Dargestellt sind 12 Parameter in 4 Kategorien (allgemeine Aktivität, Burst Struktur, Oszillation, Synchronität). (Mittelwert ± Standardfehler (SEM), Befeldungsgruppe 01- 1: n=7, Befeldungsgruppe 02- 1: n=17. ungepaarter Student'scher t - Test: * p ≤ 0.05; ** p ≤ 0.01; ***p≤ 0.001)

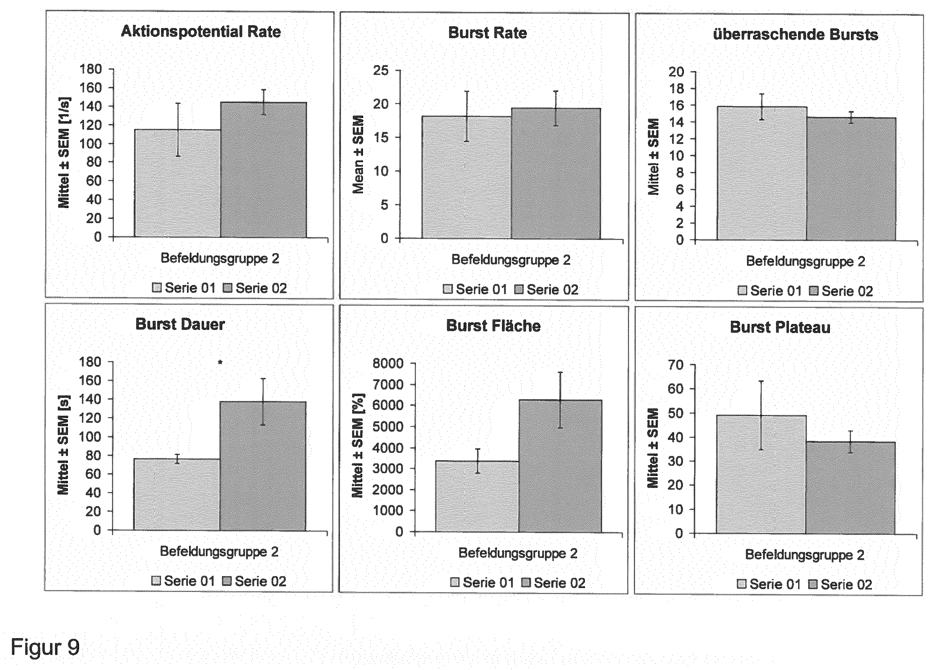

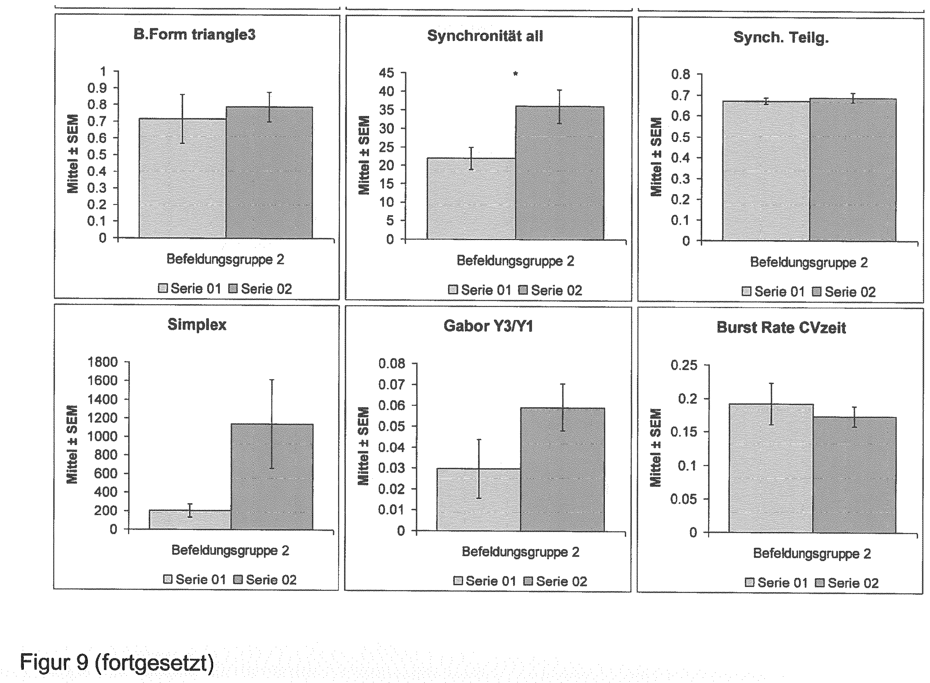

**Figur 9** zeigt die durch effektive chronische 16 Hz Befeldung induzierten Effekte (Experiment 2, Befeldungsgruppe 2, Serie 01= Leistungsstufe 6, Serie 02 = Leistungsstufe 3) auf die kortikale Netzwerkaktivität *in vitro*. Dargestellt sind 12 Parameter in 4 Kategorien (allgemeine Aktivität, Burst Struktur, Oszillation, Synchronität). (Mittelwert ± Standardfehler (SEM), Befeldungsgruppe 01- 2: n=7, Befeldungsgruppe 02- 2: n=15. ungepaarter Student'scher t - Test: * p ≤ 0.05; ** p ≤ 0.01; *** p ≤ 0.001)

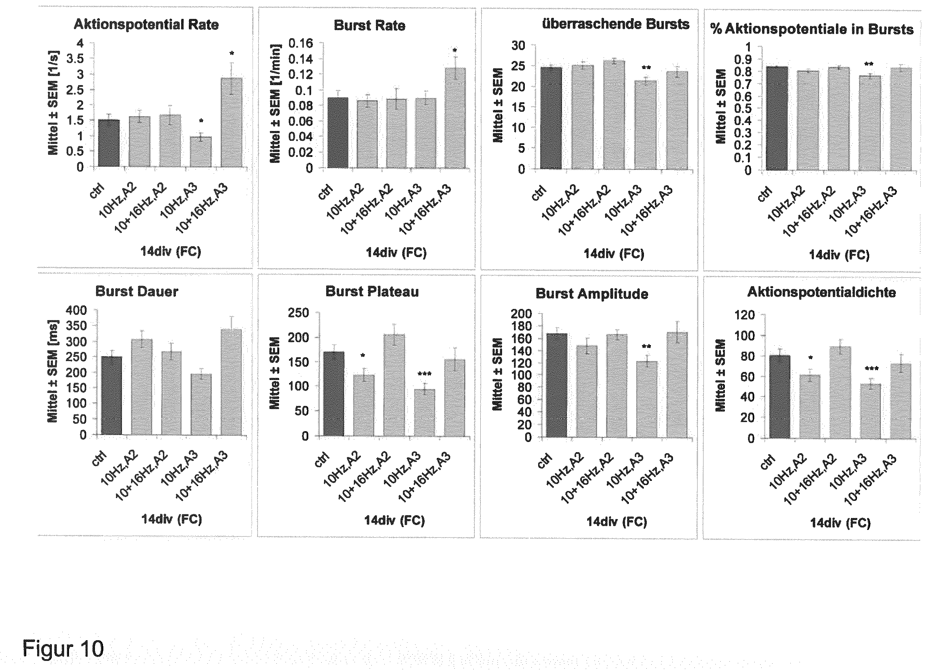

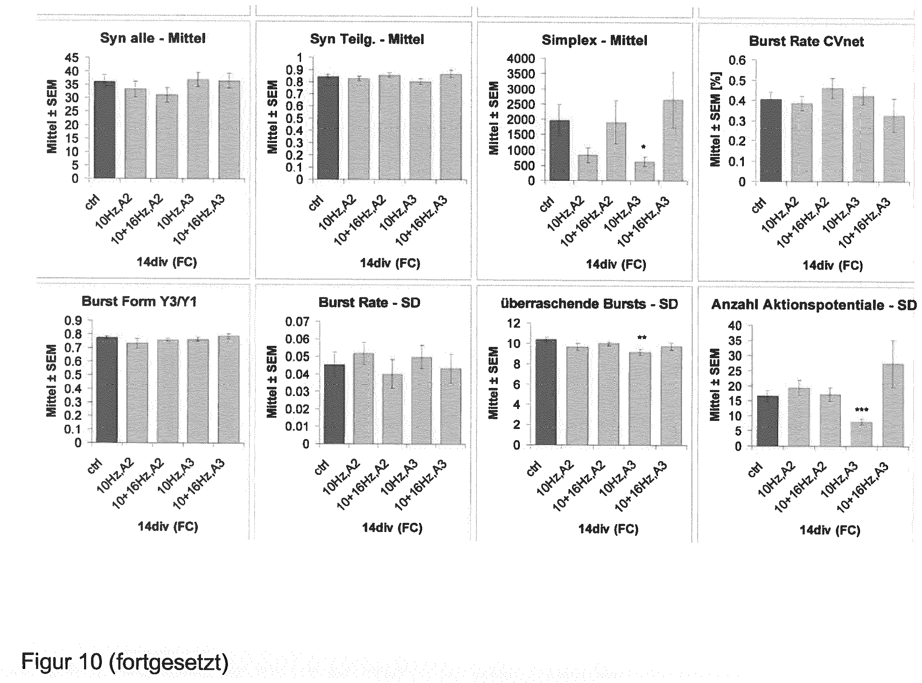

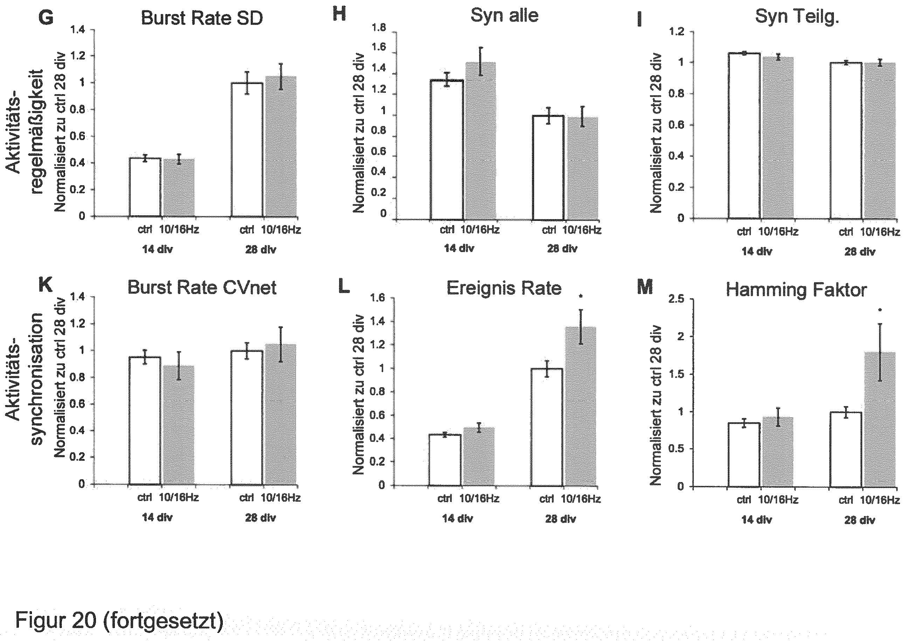

**Figur 10** zeigt die Effekte der 4 Befeldungsgruppen aus Experiment 3 auf die kortikale Netzwerkaktivität *in vitro* im Vergleich zur unbefeldeten Kontrollaktivität nach 10-tägiger (14 Tage in Kultur, engl.: days *in vitro*; div). Dargestellt sind 16 Parameter in 4 Kategorien: allgemeine Aktivität, Burst Struktur, Synchronität, Oszillation (entsprechend pro Zeile). (Mittelwert ± Standardfehler (SEM), ungepaarter Student'scher t - Test: * p ≤ 0.05; ** p ≤ 0.01; *** p ≤ 0.001, Kontrolle: Ctrl)

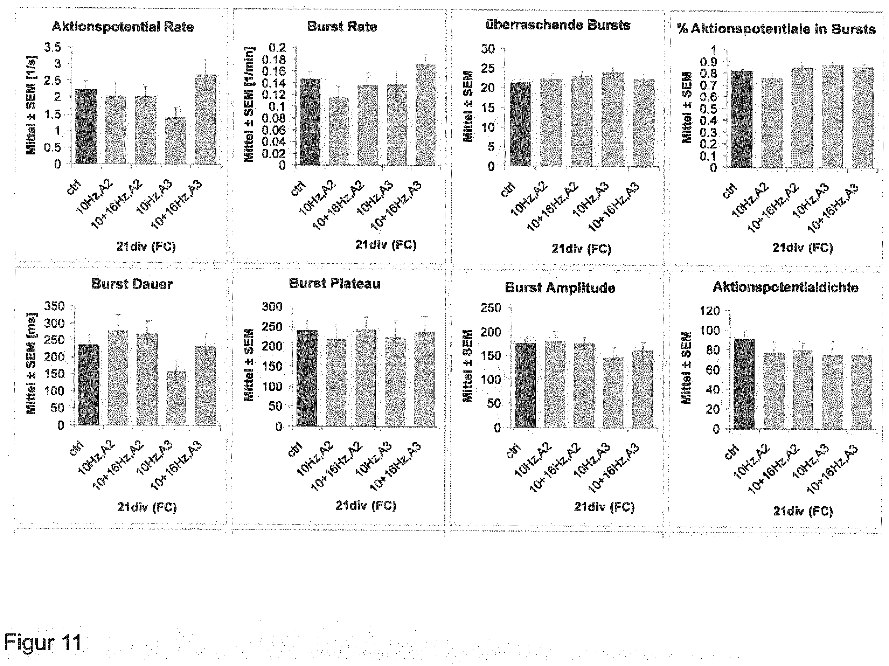

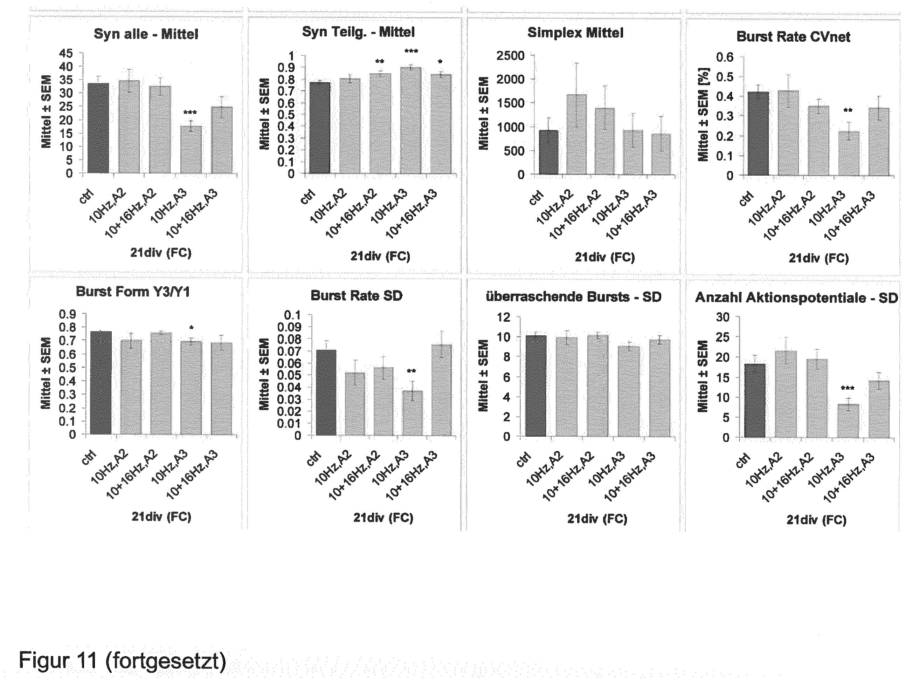

**Figur 11** zeigt die Effekte der 4 Befeldungsgruppen aus Experiment 3 auf die kortikale Netzwerkaktivität *in vitro* im Vergleich zur unbefeldeten Kontrollaktivität nach 17-tägiger (21 div). Dargestellt sind 16 Parameter in 4 Kategorien: allgemeine Aktivität, Burst Struktur, Synchronität, Oszillation (entsprechend pro Zeile). (Mittelwert ± Standardfehler (SEM), ungepaarter Student'scher t - Test: * p ≤ 0.05; ** p ≤ 0.01; *** p ≤ 0.001, Kontrolle: Ctrl)

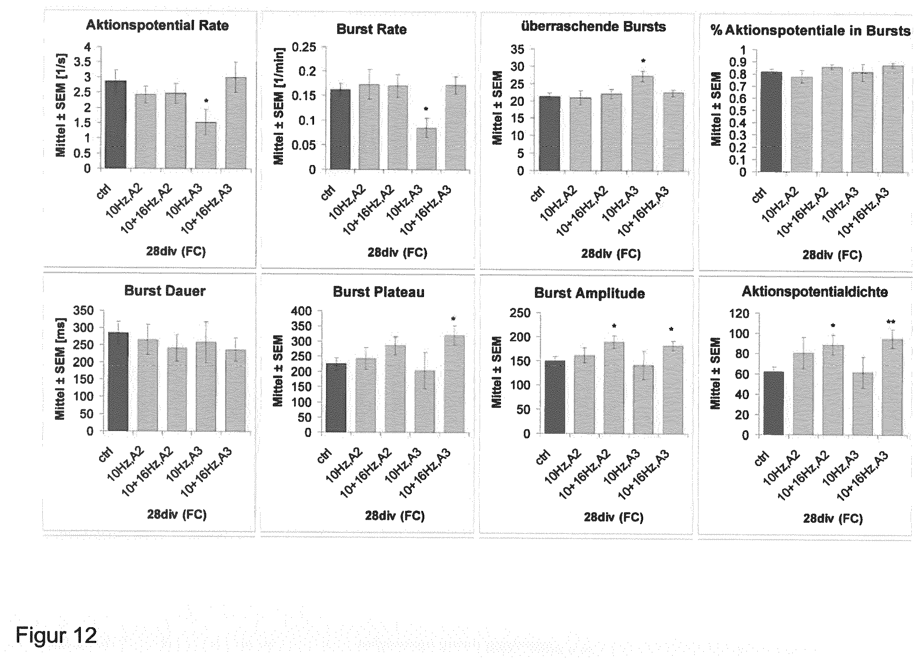

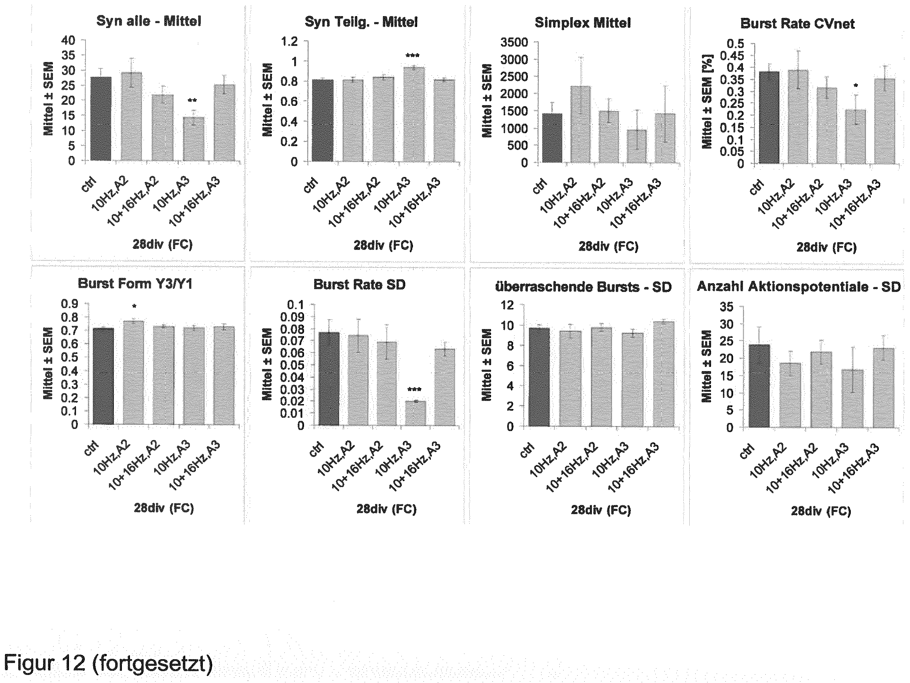

**Figur 12** zeigt die Effekte der 4 Befeldungsgruppen aus Experiment 3 auf die kortikale Netzwerkaktivität *in vitro* im Vergleich zur unbefeldeten Kontrollaktivität nach 24-tägiger (28 div). Dargestellt sind 16 Parameter in 4 Kategorien: allgemeine Aktivität, Burst Struktur, Synchronität, Oszillation (entsprechend pro Zeile). (Mittelwert ± Standardfehler (SEM), ungepaarter Student'scher t - Test: * p ≤ 0.05; ** p ≤ 0.01; *** p ≤ 0.001, Kontrolle: Ctrl)

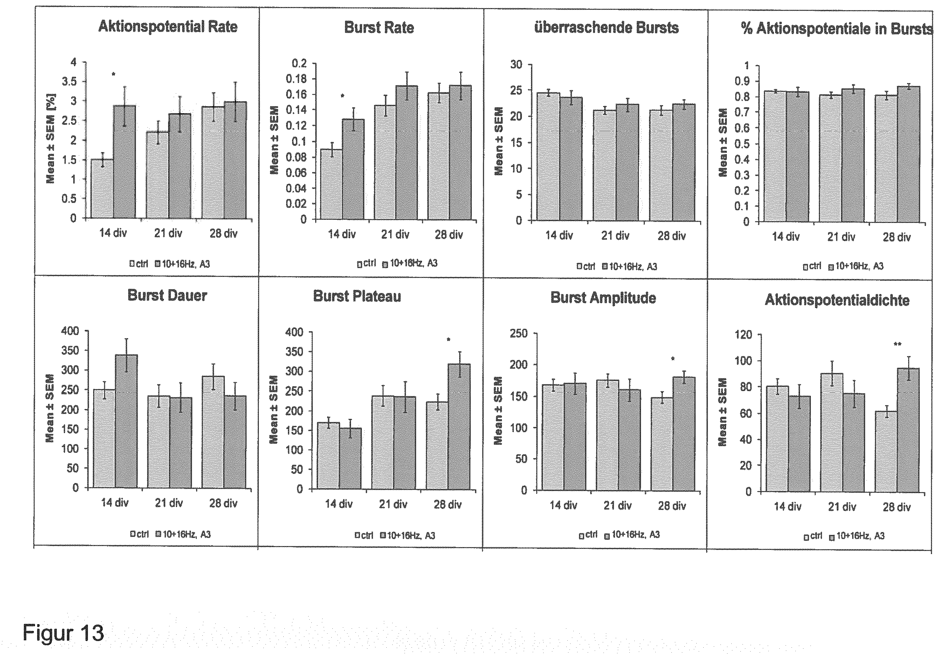

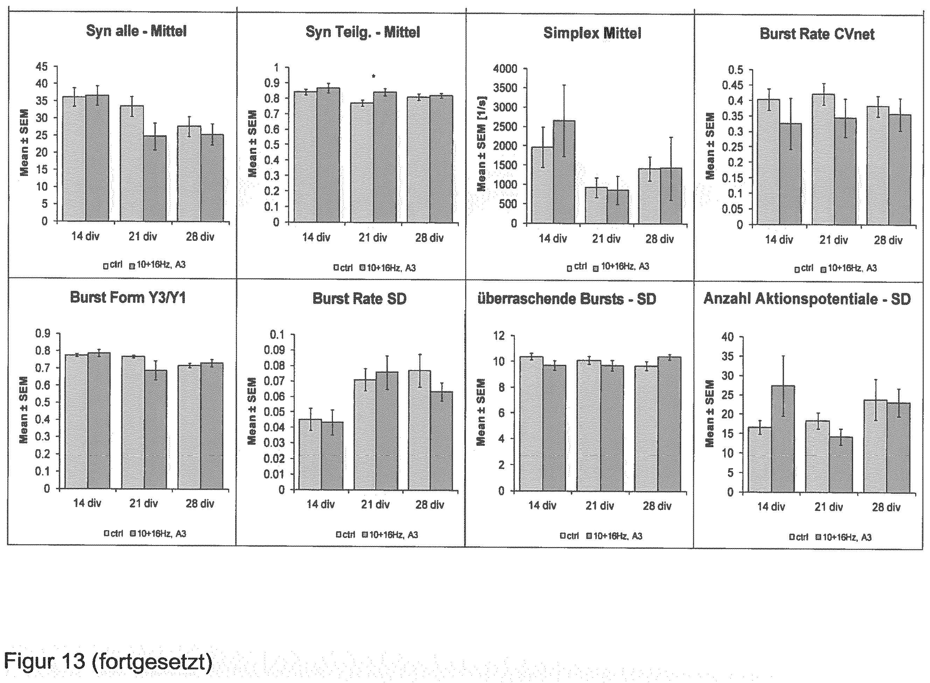

**Figur 13** zeigt die Effekte der alternierenden Befeldung mit 10 und 16 Hz im Abstand A3 auf die kortikale Netzwerkaktivität *in vitro* im Vergleich zur unbefeldeten Kontrollaktivität nach 24-tägiger (28 div) (Experiment 3). Dargestellt sind 16 Parameter in 4 Kategorien: allgemeine Aktivität, Burst Struktur, Synchronität, Oszillation (entsprechend pro Zeile). (Mittelwert ± Standardfehler (SEM), ungepaarter Student'scher t - Test: * p ≤ 0.05; ** p ≤ 0.01; *** p ≤ 0.001, Kontrolle: Ctrl)

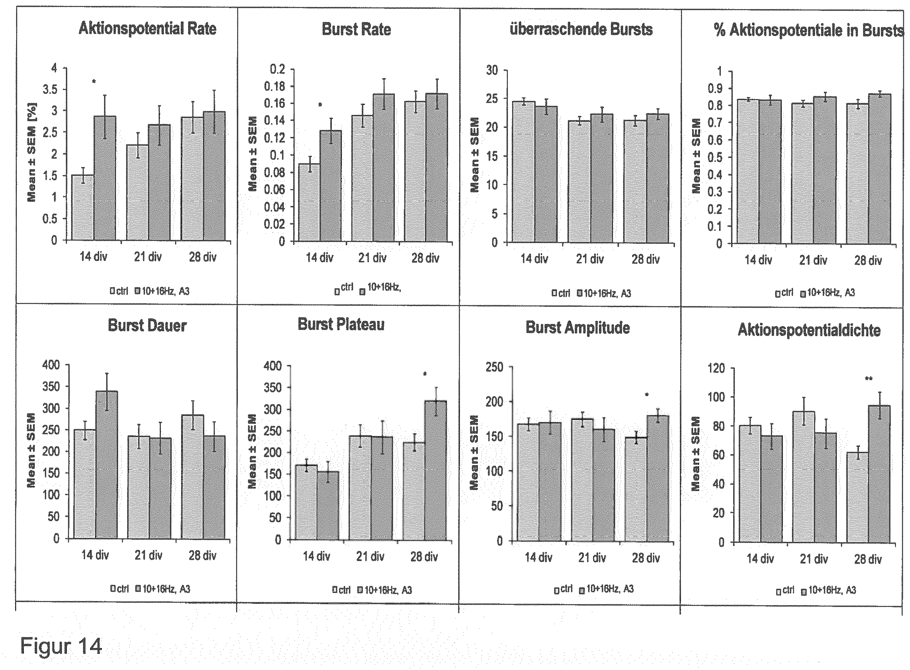

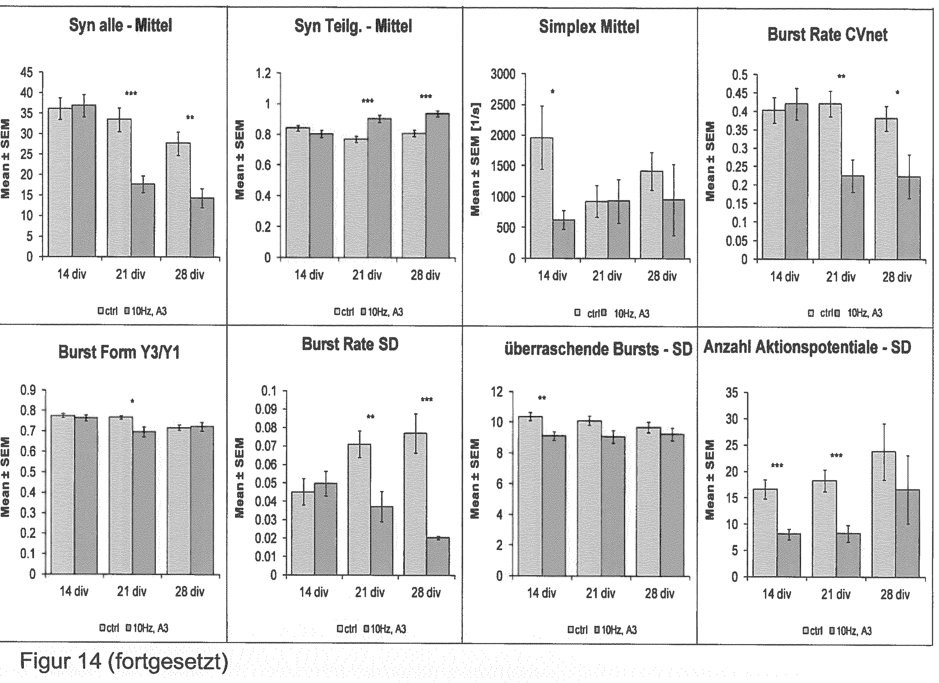

**Figur 14** zeigt die Effekte der Befeldung mit 10 Hz im Abstand A3 auf die kortikale Netzwerkaktivität *in vitro* im Vergleich zur unbefeldeten Kontrollaktivität nach 24 - tägiger (28 div) (Experiment 3). Dargestellt sind 16 Parameter in 4 Kategorien: allgemeine Aktivität, Burst Struktur, Synchronität, Oszillation (entsprechend pro Zeile). (Mittelwert ± Standardfehler (SEM), ungepaarter Student'scher t - Test: * p ≤ 0.05; ** p ≤ 0.01; *** p ≤ 0.001, Kontrolle: Ctrl)

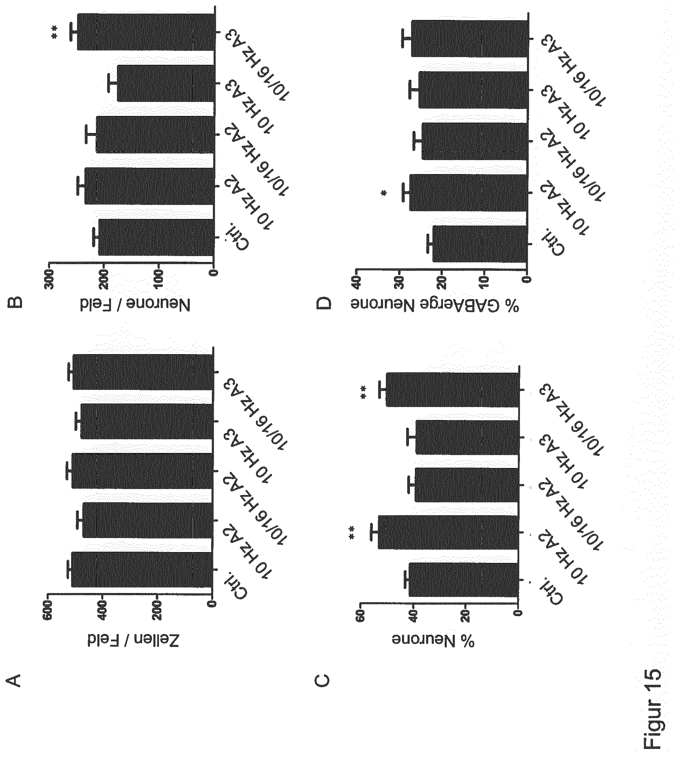

**Figur 15** zeigt die absolute Gesamtzellzahl je Bild (A), den Anteil der absoluten Neuronenzahl je Bild (B), den prozentualen Neuronenanteil an der Gesamtzellzahl (C), und den prozentualen Anteil GABAerger Neurone an der neuronalen Population (D). (zweiseitiger ungepaarter Student'scher t - Test gegen Kontrolle: * p<0.05, ** p<0.01, *** p<0.001, Kontrolle: Ctrl)

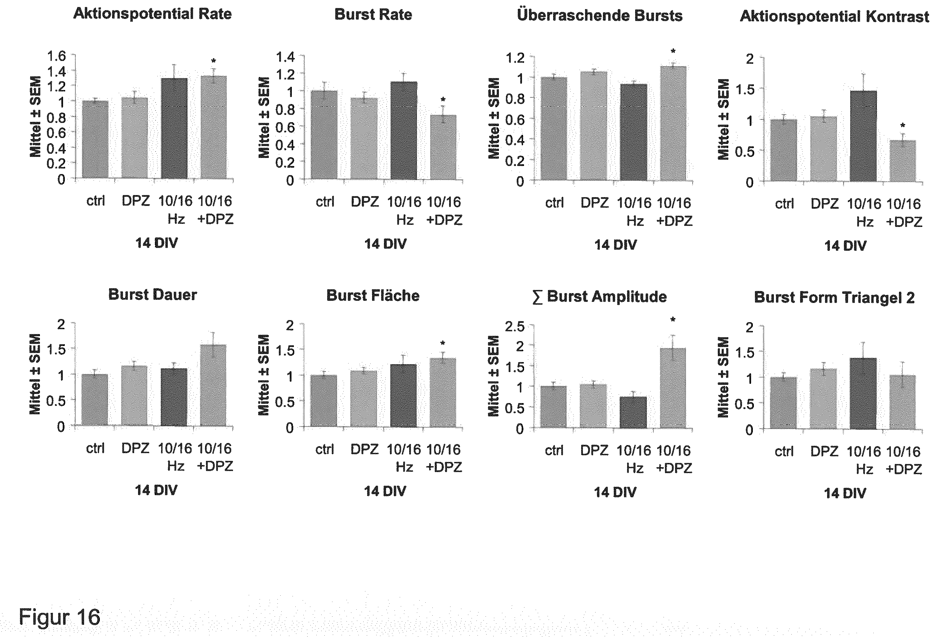

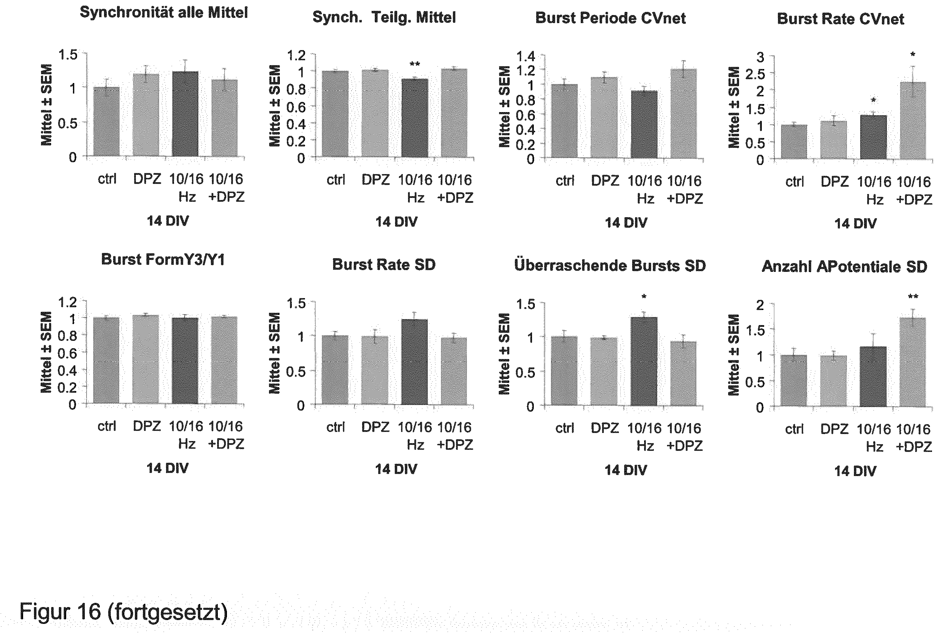

**Figur 16** zeigt den Vergleich der Effekte einer chronischen Donepezil-Behandlung, wiederholter Befeldung mit alternierenden 10/10 Hz und einer wiederholten 10/16 Hz Befeldung bei gleichzeitiger chronischer Donepezil-Behandlung auf die Netzwerkaktivität von Frontalkortex-Neuronen *in vitro* (14 DIV). Dargestellt sind 16 Merkmale, die die Netzwerkaktivität beschreiben: allgemeine Aktivität, Burst Struktur, Synchronität, Oszillation (entsprechend pro Zeile). (Mittelwert ± Standardfehler (SEM), ungepaarter Student'scher t - Test: * p ≤ 0.05; ** p ≤ 0.01; *** p ≤ 0.001)

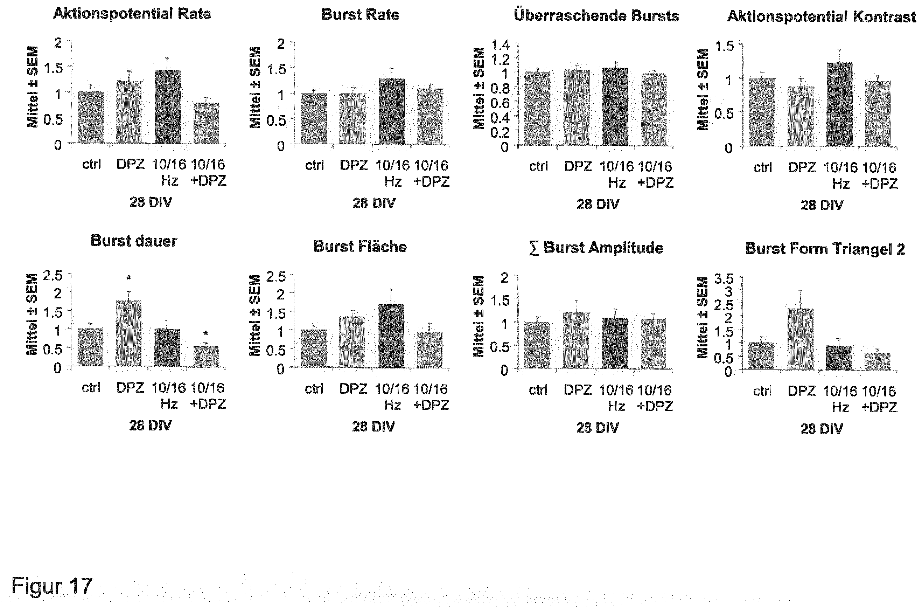

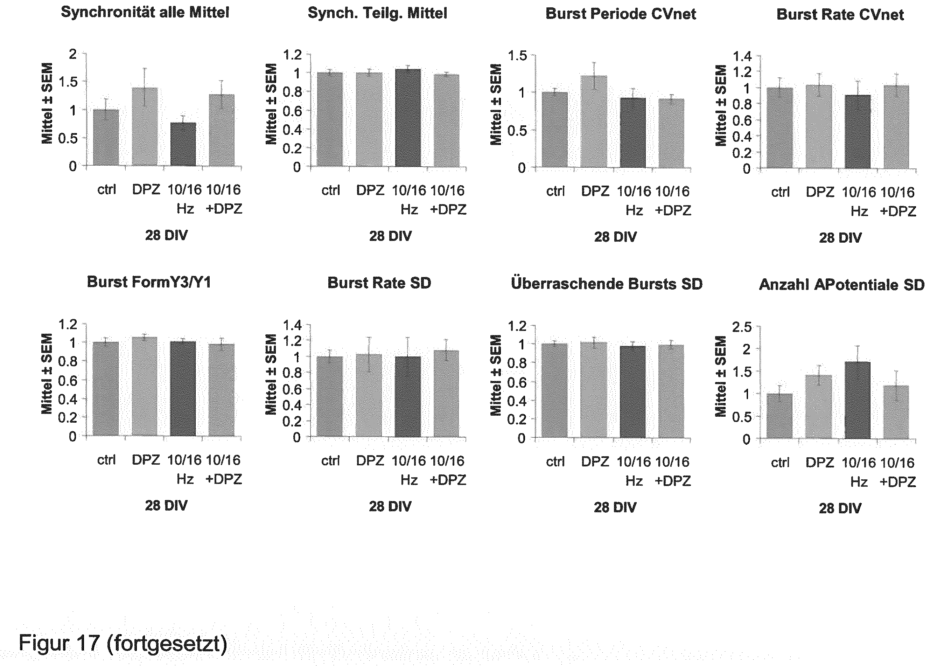

**Figur 17** zeigt den Vergleich der Effekte einer chronischen Donepezil-Behandlung, wiederholter Befeldung mit alternierenden 10/10 Hz und einer wiederholten 10/16 Hz Befeldung bei gleichzeitiger chronischer Donepezil-Behandlung auf die Netzwerkaktivität von Frontalkortex-Neuronen *in vitro* (28 DIV. Dargestellt sind 16 Merkmale, die die Netzwerkaktivität beschreiben: allgemeine Aktivität, Burst Struktur, Synchronität, Oszillation (entsprechend pro Zeile). (Mittelwert ± Standardfehler, ungepaarter Student'scher t - Test: * p ≤ 0.05; ** p ≤ 0.01; *** p ≤ 0.001)

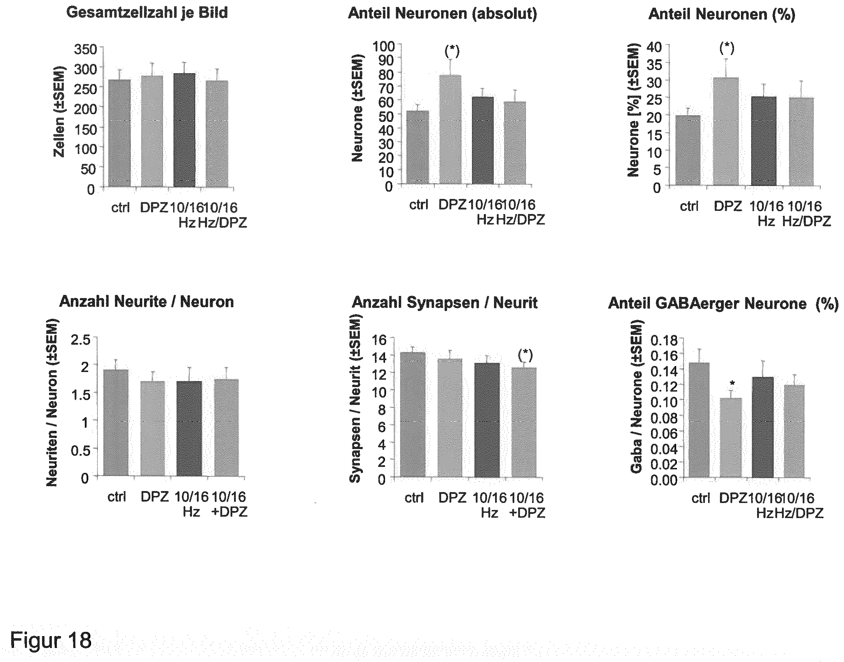

**Figur 18** zeigt den Einfluss der 3 Behandlungsgruppen Donepezil, 10+16 Hz, A3 und 10+16 Hz, A3 + DPZ auf die Morphologie der kortikalen Netzwerke nach 10-tägiger Behandlung (14 DIV), nämlich auf die absolute Gesamtzellzahl je Bild, den Anteil der absoluten Neuronenzahl, den prozentualen Neuronenanteil an der Gesamtzellzahl, die Anzahl der Neurit pro Neuron, die Anzahl der Synapsen pro Neurit, und den prozentualen Anteil GABAerger Neurone zur neuronalen Population. Zweiseitiger ungepaarter Student'scher t-Test: * p<0.05, ** p<0.01, *** p<0.001.

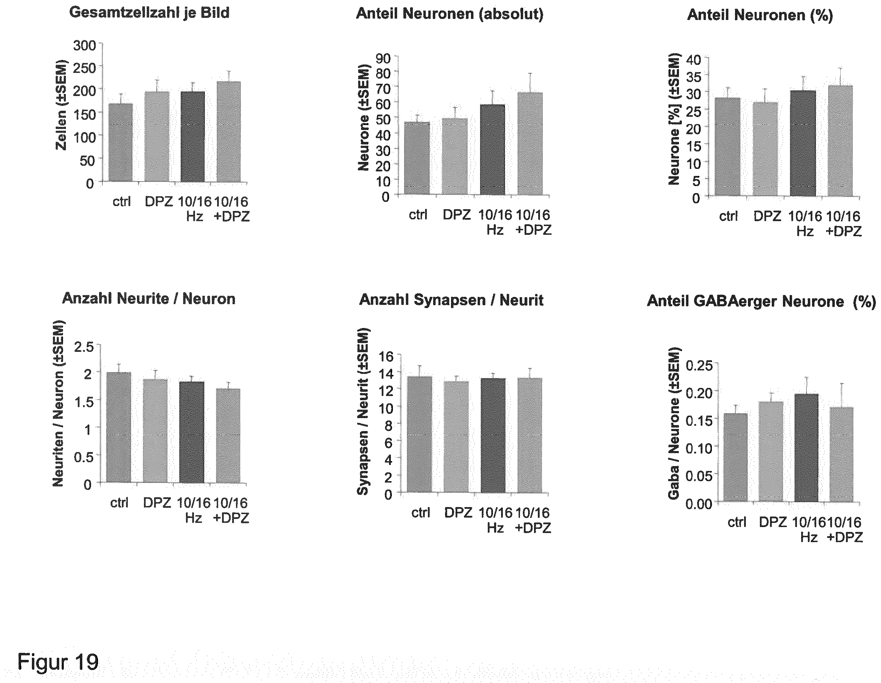

**Figur 19** zeigt den Einfluss der 3 Behandlungsgruppen Donepezil, 10+16 Hz, A3 und 10+16 Hz, A3 + DPZ auf die Morphologie der kortikalen Netzwerke nach 24-tägiger Behandlung (28 DIV), nämlich auf die absolute Gesamtzellzahl je Bild, den Anteil der absoluten Neuronenzahl, den prozentualen Neuronenanteil an der Gesamtzellzahl, die Anzahl der Neurit pro Neuron, die Anzahl der Synapsen pro Neurit, und F) den prozentualen Anteil GABAerger Neurone zur neuronalen Population. ungepaarter Student'scher t - Test: * p<0.05, ** p<0.01, *** p<0.001.

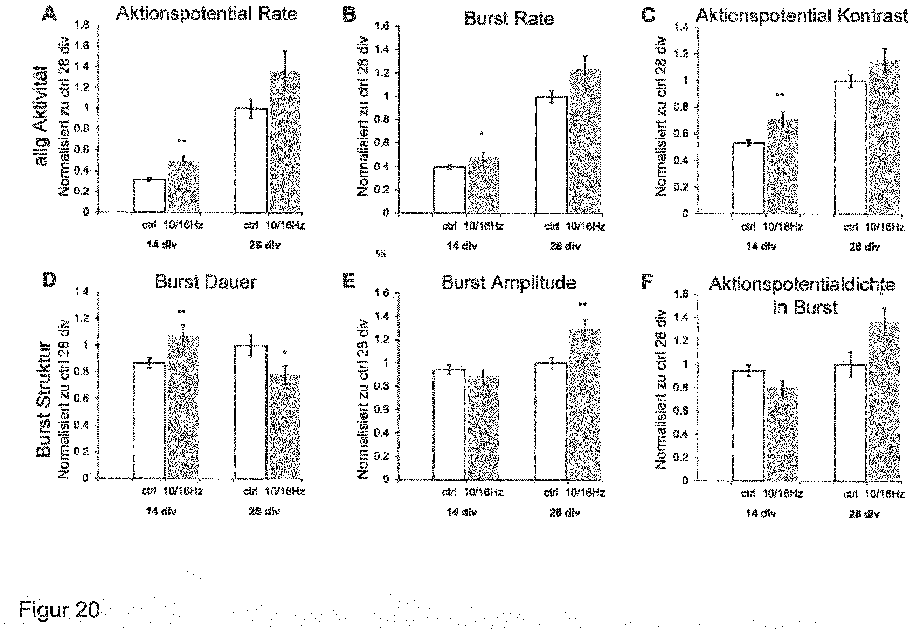

**Figur 20** zeigt den Einfluss eines 150 MHz elektromagnetischen Feldes, das mit 10 und 16 Hz gepulst wurde, auf die Differenzierung der kortikalen Netzwerkaktivität. Gezeigt sind 12 Parameter, die die Aktivität beschreiben. Sie zeigen die Veränderungen in vier Aktivitätskategorien, allgemeine Aktivität (A - C), Burst Struktur (D - F), Synchronisation (G - I) und Oszillation (K - M). (Mittelwert ± Standardfehler, ungepaarter Student'scher t - Test: * p ≤ 0.05; ** p ≤ 0.01; *** p ≤ 0.001)

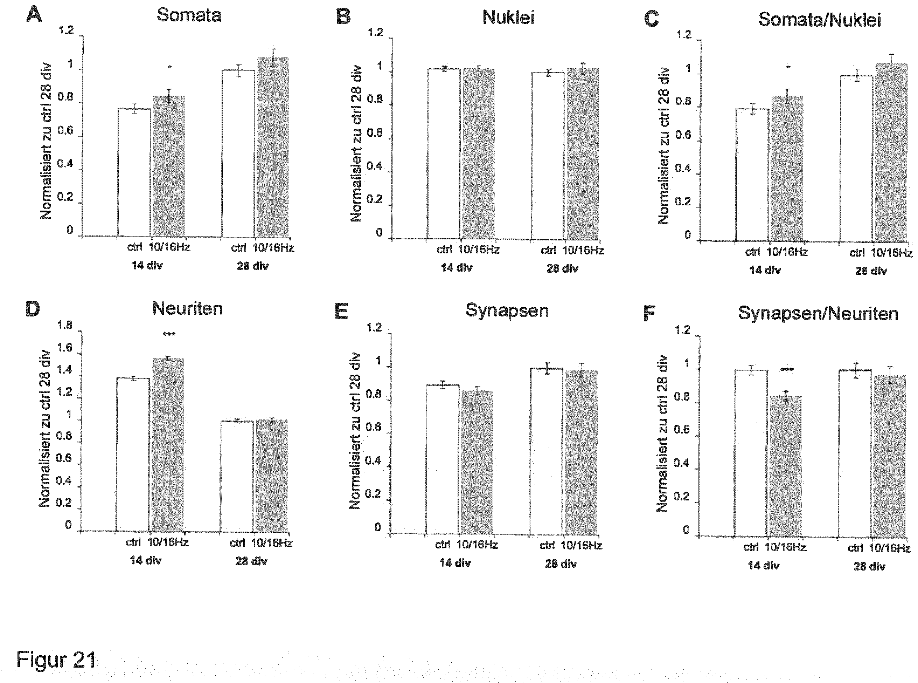

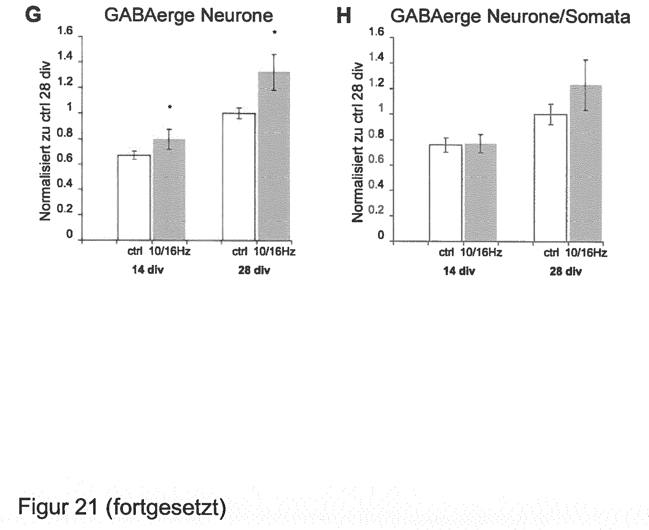

**Figur 21** zeigt die Effekte eines mit 10 und 16 Hz modulierten 150 MHz elektromagnetischen Feldes auf die Entwicklung der Morphologie des kortikalen Netzwerks. Die Daten wurden durch halb-automatische Quantifizierung gewonnen. (Mittelwert ± Standardfehler, ungepaarter Student'scher t - Test: * p ≤ 0.05; ** p ≤ 0.01; *** p ≤ 0.001)

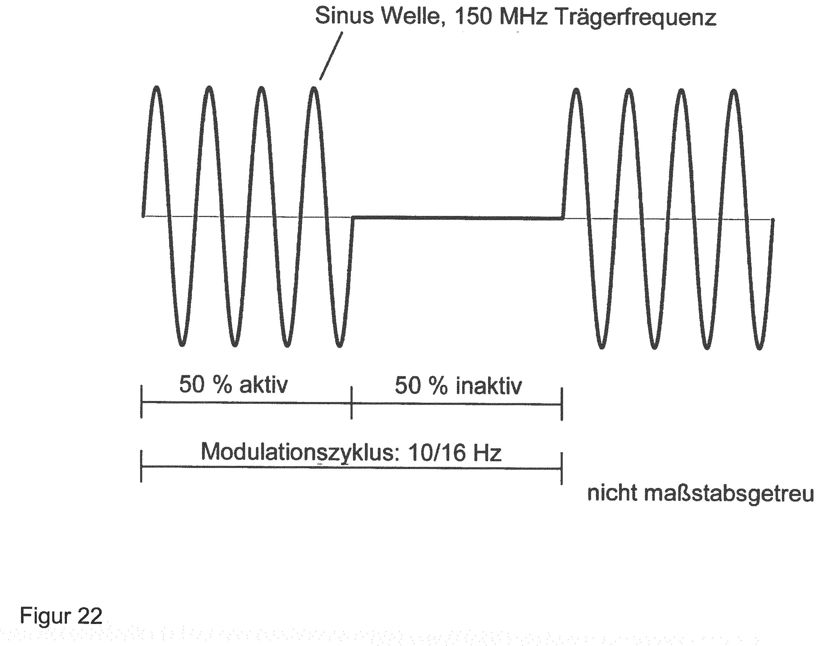

**Figur 22** zeigt die schematische Darstellung der Wellenform eines elektromagnetischen Feldes mit einer Trägerfrequenz (150 MHz), die wiederholt mit 10 und 16 Hz moduliert wurde (typischerweise 100 und 62,5 ms).

### Detaillierte Beschreibung der Erfindung

Die Erfindung betrifft ein *in vitro* Verfahren zur Förderung der Entwicklung von Neuronen, umfassend die Schritte Kultivieren der Neurone aus neuronalen Vorläuferzellen, und Befelden der neuronalen Vorläuferzellen und/oder der Neurone während des Kultivierens mit einer Hochfrequenzschwingung, die eine Frequenz von 100 bis 200 MHz aufweist, bei dem die Hochfrequenzschwingung durch eine Niederfrequenzschwingung, die eine Frequenz von 5 bis 20 Hz aufweist, moduliert wird.

Der Begriff "Vorläuferzellen" bezeichnet Zellen, welche die Fähigkeit besitzen sich zu teilen. Die Teilung erfolgt entweder proliferativ symmetrisch, wobei zwei Vorläuferzellen gebildet werden, asymmetrisch, wobei eine Vorläuferzelle und eine post-mitotische Zelle gebildet werden, oder differenzierend symmetrisch, wobei zwei post-mitotische Zellen gebildet werden. Der Begriff "neuronale Vorläuferzellen" bezeichnet Vorläuferzellen, welche die Fähigkeit besitzen direkt oder indirekt Neurone zu bilden. Hierzu zählen beispielsweise neurale Stammzellen, welche in der Lage sind Neurone, Glia und Oligodendrozyten zu bilden, als auch Neuroepithelzellen, die bereits auf die Bildung von Neuronen determiniert sind.

Das Nervensystem von Wirbeltieren, beispielsweise des Menschen, umfasst viele verschiedene individuelle Arten von Neuronen (Nervenzellen) mit unterschiedlichen Funktionen. Dennoch verläuft die Entwicklung aller Neurone (sog. Neurogenese) ähnlich, und die grundlegenden Regulationsmechanismen der Neurogenese unterschiedlicher Neurone sind dieselben. So können beispielsweise Neuroepithelzellen des Kortex nacheinander Neurone unterschiedlicher kortikaler Schichten mit individuellen Funktionen bilden. Die verschiedenen Arten von Neuronen können auf unterschiedliche Weise klassifiziert werden, beispielsweise an Hand der von ihnen verwendeten Neurotransmitter, z.B. Glutamat oder gamma-Aminobuttersäure (GABA), oder nach Art der von ihnen ausgelösten Reaktionen, z.B. erregend, hemmend oder modulierend. Außerdem werden Neurone auch nach der Richtung des Informationsflusses unterschieden, nämlich in afferente Neurone, die Information zum Gehirn transportieren, in efferent Neurone, die Information vom Gehirn in die Organe bringen, und Interneurone, die Information zwischen einzelnen Neuronen austauschen.

Der Begriff "Entwicklung von Neuronen" bezeichnet sowohl die Bildung neuer Neurone durch Vorläuferzellen, als auch die Differenzierung und Reifung der Zellen zur vollständigen Funktionalität. Entsteht bei der Teilung einer neuronalen Vorläuferzelle eine post-mitotische Zelle, steht deren weiteres Schicksal bereits weitgehend fest. Soll aus der Zelle ein Neuron werden, so entwickeln sich je nach Art des Neurons ein Axon, wenige oder mehrere Dendriten und Synapsen. Anschließend bilden die Neurone Verbindungen zu einander und zu benachbarten Glia-Zellen, so dass ein neuronales Netzwerk entsteht.

Gemäß dem erfindungsgemäßen Verfahren werden neuronale Vorläuferzellen unter Bedingungen kultiviert, welche die Bildung von Neuronen ermöglichen. Hierzu werden die Vorläuferzellen in der Regel auf Kulturschalen, die mit Poly-D-Lysin und/oder Laminin beschichtet sind, ausgebracht. Dem Kulturmedium werden häufig Rinder- oder Pferdeserum beigegeben, um die Zellen in einer möglichst physiologischen Umgebung zu halten. In Abhängigkeit vom Alter, der Entwicklungsstufe und der Herkunft neuronaler Vorläuferzellen, können unterschiedliche Kulturbedingungen vorteilhaft sein.

Interessanterweise wurde festgestellt, dass eine Befeldung einer Kultur die neuronale Vorläuferzellen und gegebenenfalls auch neu gebildete Neurone enthält, die Entwicklung der Neuronen positiv beeinflusst. Der Begriff" Befeldung" bezeichnet hierbei, dass ein Gewebe oder Zellen elektromagnetischen Wellen ausgesetzt werden, wobei es sich bei den elektromagnetischen Wellen um eine Hochfrequenzschwingung handelt, die durch eine Niederfrequenzschwingung moduliert ist. Ohne an eine bestimmte Theorie gebunden zu sein, wird davon ausgegangen, dass dieser Effekt auf eine Interaktion der elektromagnetischen Wellen der Befeldung mit den elektromagnetischen Wellen der Zellen zurückzuführen ist. Es wurde festgestellt, dass es in Zellen und Geweben elektromagnetische Strahlung gibt, deren Intensität in Größenordnungen von einigen 100 bis 1000 Photonen pro Sekunde und cm² liegt. Die Energien der Photonen erstrecken sich auf den Wellenbereich von 800 bis etwa 350 nm mit einem Maximum bei ca. 550 nm. Dementsprechend kommt es bei einer Bestrahlung von Gewebe mit einer niederfrequenzmodulierten hochfrequenten Grundfrequenz von 100 bis 200 MHz, zu biologischen Reaktionen der befeldeten Zellen.

Werden neuronale Vorläuferzellen gemäß des erfindungsgemäßen Verfahrens befeldet, steigt die allgemeine Aktivität der in Kultur gebildeten Neurone. Dies ist ein Hinweis darauf, dass es sich bei den gebildeten Neuronen um funktionelle Nervenzellen handelt, die neuronale Netzwerke bilden können. Zudem wurde beobachtet, dass durch die Befeldung die Anzahl der Neurone gegenüber nicht neuronalen Zellen steigt. Durch die Befeldung wird somit eine erhöhte Reinheit der Kultur erreicht. Darüber hinaus führte die Befeldung auch zu einer beschleunigten Entwicklung der Neurone, wodurch die Kultivierungszeit verkürzt wird. Dadurch sinkt die Gefahr einer Kontamination der Kultur, und es lassen sich schneller funktionelle Neurone gewinnen.

Die Befeldung ist zudem gut geeignet, die Einwicklung von Neuronen unter möglichst physiologischen Bedingungen zu fördern, da sie von den übrigen Kulturbedingungen unabhängig ausgeführt werden kann. Darüber hinaus ist auch eine unmittelbare Anwendung der Befeldung am Patienten möglich, denn die Befeldung kann ohne invasiven Eingriff ausgeführt werden. Somit kann durch die Befeldung die Neurogenese endogener neuronaler Vorläuferzellen stimuliert werden.

Bei der Befeldung werden die Zellen einem modulierten Wellenzug ausgesetzt. Die Hochfrequenzschwingung dient hierbei als Trägerfrequenz, die durch eine Niederfrequenzschwingung (Modulationsfrequenz) moduliert wird **(****Figur 22**). Die Befeldung erfolgt mittels einer Bestrahlungsvorrichtung, die eine Hochfrequenzoszillationsstufe, eine Niederfrequenzoszillationsstufe und eine Modulationseinheit aufweist. Indem der Ausgang der Hochfrequenzoszillationsstufe und der Ausgang der Niederfrequenzoszillationsstufe der Modulationseinheit zugefügt werden, wird ein modulierter Wellenzug gebildet. Geeignete Bestrahlungsvorrichtungen sind beispielsweise in folgenden Dokumenten beschrieben: EP 0 136 530 B1, DE 101 00 385 A1, DE 10 2005 014 023 A1 und EP 2 153 869 A1.

In einer bevorzugten Ausführungsform weist die Hochfrequenzschwingung eine Frequenz von 130 bis 170 MHz, bevorzugt von 150 MHz auf. Für die Behandlung von Gewebe und lebenden Zellen hat sich eine Hochfrequenzschwingung von 150 MHz als vorteilhaft erwiesen. Das generierte Signal kann besonders gut mit den Zellen interagieren und dementsprechend starke und reproduzierbare zelluläre Reaktionen hervorrufen.

In einer bevorzugten Ausführungsform weist die Niederfrequenzschwingung eine Frequenz von 10 bis 20 Hz, bevorzugt von 10 Hz oder 16 Hz auf. Die Befeldung mit 10 bzw. 16 Hz zeigte einen signifikanten Einfluss auf die Entwicklung von Neuronen. Insbesondere führte sie zu einer vermehrten Bildung von Neuronen auf Kosten glialer Zellen und erhöhte zudem die Aktivität der Neurone, wodurch eine erhöhte Konnektivität indiziert wird.

In einer bevorzugten Ausführungsform wird die Hochfrequenzschwingung abwechselnd durch eine Niederfrequenzschwingung mit einer Frequenz von 10 Hz und 16 Hz moduliert. Bei einer Befeldung der neuronalen Vorläuferzellen mit alternierender Frequenzmodulation konnte bereits nach 10 Tagen in Kultur eine erhöhte Aktivität der gebildeten Neurone nachgewiesen werden. Dies zeigt, dass diese Befeldung zu einer beschleunigten Differenzierung der gebildeten Neurone führt, wodurch die Gesamtdauer der Kultur verringert werden kann.

In einer bevorzugten Ausführungsform werden die neuronalen Vorläuferzellen durch eine Bestrahlungsvorrichtung befeldet und in einem Abstand von 1 bis 100 mm, bevorzugt 30 bis 100 mm, ferner bevorzugt 50 bis 100 mm von der Bestrahlungsvorrichtung angeordnet. Durch den Abstand der Zellen von der Schwingungsquelle wird beeinflusst wie stark die Zellen der Befeldung ausgesetzt sind. Es hat sich gezeigt, dass der Abstand der Zellen von der Strahlungsquelle die Wirkung der Befeldung beeinflusst, wobei im allgemeinen bei einer schwächeren Niederfrequenz, von beispielsweise 5 bis 10 Hz, ein näherer Abstand, beispielsweise 30 bis 50 mm, bevorzugt ist. Bei einer stärkeren Niederfrequenz, beispielsweise 15 bis 20 Hz, führt hingegen ein größerer Abstand, beispielsweise 80 bis 100 mm, zu besseren Ergebnissen.

In einer bevorzugten Ausführungsform sind die neuronalen Vorläuferzellen während der Befeldung einer magnetischen Feldstärke von 0,05 bis 1,5 Pikotesla (pT), bevorzugt 0,1 bis 0,5 pT, ferner bevorzugt von 0,125 pT ausgesetzt. Bei der Befeldung werden die neuronalen Vorläuferzellen einer magnetischen Feldstärke ausgesetzt, deren Stärke den Effekt der Befeldung auf die Zellen beeinflussen kann. Es wurde gefunden, dass für die Förderung der Entwicklung von Neuronen eine Feldstärke von 0,05 bis 1,5 pT bevorzugt ist. Die effektivste Bildung und Differenzierung von Neuronen wurde bei einer Befeldung mit einer magnetischen Feldstärke von 0,125 pT beobachtet. Dies führte zu einer erhöhten Anzahl von Neuronen und förderte spezifisch die Bildung von Interneuronen.

In einer bevorzugten Ausführungsform erfolgt die Befeldung mit einer maximalen Leistungsabgabe durch die Bestrahlungsvorrichtung von 0,055 bis 1,85 mW, bevorzugt 0,12 bis 1,85 mW, ferner bevorzugt von 0,12 mW. Diese geringe Leistung ist bereits ausreichend, um die Entwicklung der Neurone zu fördern. Gleichzeitig wird jedoch ausgeschlossen, dass die Zellen durch die Strahlung, der sie ausgesetzt, sind Schaden nehmen. Insbesondere kommt es nicht zu unerwünschtem thermischen Stress.

In einer bevorzugten Ausführungsform werden die neuronalen Vorläuferzellen einbis viermal pro Woche, bevorzugt zweimal pro Woche befeldet. Die Kultivierung neuronaler Vorläuferzellen erfolgt unter möglichst konstanten Bedingungen, um eine Kontaminierung zu vermeiden und eine stabile Proliferation und Differenzierung zu gewährleisten. Hierzu sollte die Kultur unter möglichst gleichbleibenden Bedingungen gehalten werden, insbesondere nur zum notwendigen Mediumwechsel aus dem Inkubator entfernt und geöffnet werden. Im Gegensatz zur Zugabe von Wachstumsfaktoren oder Signalmolekülen, für die die Kultur jeweils aus dem Inkubator entfernt werden muss, kann die Befeldung ohne Unterbrechung der Kultivierung erfolgen. Zudem zeigt sich der positive Effekt des Verfahrens bereits bei einer zweimaligen Befeldung pro Woche, so dass das Verfahren wenig arbeitsintensiv ist.

In einer bevorzugten Ausführungsform werden die neuronalen Vorläuferzellen jeweils für mindestens 30 Minuten, bevorzugt für 30 bis 60 Minuten, ferner bevorzugt für 60 Minuten befeldet. Untersuchungen haben gezeigt, dass eine Befeldungsdauer von 60 Minuten pro Befeldungsvorgang ausreicht, um die Entwicklung von Neuronen zu fördern. Bei der Verwendung einer Befeldung mit wechselnden Niedrigfrequenzschwingungen sollte die Befeldungsdauer daher ca. 30 Minuten pro Frequenz betragen.

In einer bevorzugten Ausführungsform umfasst das Verfahren ferner den Schritt Zugeben eines Antidementivas, bevorzugt eines Acetylcholinesterasehemmers zu den Zellen. Der Begriff "Zugeben eines Antidementivas" wie hier verwendet umfasst sowohl die einfache als auch wiederholte Zuführung eines Antidementivas, beispielsweise die Kultivierung von Zellen in Gegenwart eines Antidementivas. Als Antidementiva werden Pharmaka bezeichnet, die zur Behandlung dementieller Syndrome (Demenzen) verwendet werden. Demenzen sind durch eine qualitative und quantitative Abnahme der Hirnleistung charakterisiert, die unterschiedliche Ursachen haben kann. Die häufigste Ursache von Demenzen ist die Alzheimer-Erkrankung. Demenzen können aber auch durch Hirninfarkte (z.B. Multifarktdemenz), genetische bedingte neurogenerative Erkrankungen oder durch Hirntraumata, Hirntumore oder Intoxinationen (z.B. durch Alkohol) verursacht bzw. gefördert werden. Zur Behandlung von Demenzen werden verschiedene Pharmaka eingesetzt, unter anderem Nootropika, Acetylcholinesterasehemmer und Nichtkompetitive NMDA-Antagonisten. Bekannte Acetylcholinesterasehemmer sind Donepezil, Tacrin, Galantamin und Rivastigmin. Die Wirkungen von Acetylcholinesterasehemmern konnte auch in vitro Kulturen von Neuronen beobachtet werden. So führt die Zugabe von Donepezil zu neuronalen Kulturen zu einem Anstieg der allgemeinen Aktivität nach 28 Tagen Kultur in vitro. Interessanterweise konnte die neuronale Aktivität und insbesondere die neuronale Entwicklung durch die Kombination der Zugabe von Donepezil und der Befeldung der Zellen deutlich gesteigert werden.

In einer bevorzugten Ausführungsform werden die neuronalen Vorläuferzellen mindestens während der ersten 28 Tage in Kultur, bevorzugt mindestens während der ersten 10 Tage in Kultur befeldet. Am Anfang der Kultivierung teilen sich die Vorläuferzellen überwiegend asymmetrisch, das heißt unter Bildung einer Vorläuferzelle und einer post-mitotischen Zelle, die sich selbst nicht mehr teilen kann und in ihrer Identität festgelegt ist. Bei der post-mitotischen Zelle kann es sich um ein Neuron, einen Astrozyten oder einen Oligodendrozyten handeln. Indem die Befeldung am Anfang der Kultur eingesetzt wird, kommt es zu einer Identitätsverschiebung zugunsten der Neurone, so dass die Bildung von Neuronen gegenüber der Bildung von Glia Zellen bevorzugt wird. Mit Fortschreiten der Kultur nimmt die Anzahl der asymmetrischen Teilungen ab und es kommt überwiegend zu Teilungen, bei denen zwei post-mitotische Zellen gebildet werden. Auch hier führt die Befeldung zu einer Verschiebung zugunsten der Bildung von Neuronen. Zusätzlich wurde beobachtet, dass die Befeldung die Differenzierung der Neurone fördert, so dass bereits innerhalb von 10 Tagen aktive Neurone gebildet werden.

In einer bevorzugten Ausführungsform sind die neuronalen Zellen Interneurone. Interneurone haben eine wichtige Rolle in der Verarbeitung von neuronaler Information, da sie Signale zwischen Neuronen weitergeben, filtern und synchronisieren. Sowohl für medizinische Anwendungen, als auch für wissenschaftliche Untersuchungen ist es vorteilhaft, weitgehend reine Kulturen zu verwenden. Sie ermöglichen zuverlässige und reproduzierbare Ergebnisse. Durch die Befeldung wird einerseits die Bildung von Neuronen gegenüber Glia Zellen gefördert. Zudem verschiebt sich die Differenzierung der Neurone hin zu Interneuronen, wodurch Kulturen mit einem besonders hohen Anteil an Interneuronen erhalten werden. Diese können verwendet werden, um die Funktion und Arbeitsweise von Interneuronen zu untersuchen. Zudem kann bei Transplantationen die Befeldung verwendet werden, um Kulturen mit einem hohen Anteil von Interneuronen zur Verfügung zu stellen. Die vermehrte Bildung von Interneuronen war insbesondere bei zwei Ausführungsformen besonders prominent, nämlich bei Verwendung einer Modulationsfrequenz von 10 Hz oder 16 Hz, einer magnetischen Feldstärke von 0,125 pT und einem Abstand der Kulturen von mindestens 30 mm von der Befeldungsquelle, sowie bei Verwendung einer alternierenden Modulationsfrequenz von 10 und 16 Hz, einer magnetischen Feldstärke von 0,125 pT und einem Abstand der Kulturen von der Befeldungsquelle von mindestens 80 mm.

In einer besonders bevorzugten Ausführungsform weist die Hochfrequenzschwingung eine Frequenz von 150 mHz und die Niederfrequenzschwingung eine Frequenz von 16 Hz auf. Zudem beträgt die magnetische Feldstärke 1 pT und der Abstand der neuronalen Vorläuferzellen von der Bestrahlungsvorrichtung 50 bis 100 mm. Eine Befeldung neuronaler Vorläuferzellen unter diesen Bedingungen führt zu einer erhöhten Aktivität der neuronalen Zellen, das heißt zur vermehrten Bildung von funktionsfähiger Neurone, die in der Lage sind neuronale Netzwerke zu bilden und sich in bestehende Netzwerke zu integrieren.

In einer besonders bevorzugten Ausführungsform weist die Hochfrequenzschwingung eine Frequenz von 150 mHz und die Niederfrequenzschwingung eine Frequenz von 16 Hz auf. Zudem beträgt die magische Feldstärke 0,125 pT und die neuronalen Vorläuferzellen sind in einem Abstand von 30 bis 100 mm, bevorzugt von 30 bis 50 mm, von der Bestrahlungsvorrichtung entfernt angeordnet. Durch eine Befeldung unter diesen Bedingungen wird die Anzahl der gebildeten Neurone im Vergleich zu nicht neuronalen Zellen (Glia und Oligodendrozyten) gefördert, wodurch reinere Kulturen erhalten werden. Außerdem kommt es zur erhöhten Bildung von Interneuronen.

In einer besonders bevorzugten Ausführungsform weist die Hochfrequenzschwingung eine Frequenz von 150 mHz und die Niedrigfrequenzschwingung eine Frequenz von 10 Hz auf. Zudem beträgt die magnetische Feldstärke 0,125 pT und die neuronalen Vorläuferzellen sind in einem Abstand von 30 bis 100 mm, bevorzugt von 40 bis 80 mm, von der Bestrahlungsvorrichtung angeordnet. Auch unter diesen Bedingungen führt die Befeldung zur bevorzugten Bildung von Neuronen, insbesondere von Interneuronen.

In einer bevorzugten Ausführungsform weist die Hochfrequenzschwingung eine Frequenz von 150 MHz und die Niederfrequenzschwingung alterierend eine Frequenz von 10 Hz und 16 Hz auf. Zudem beträgt die magnetische Feldstärke 0,125 pT und die neuronalen Vorläuferzellen sind in einem Abstand von 50 bis 100 mm, bevorzugt von 80 bis 100 mm, von der Bestrahlungsvorrichtung angeordnet. Eine Befeldung unter diesen Bedingungen führt neben einer bevorzugten Bildung von Neuronen zu einer schnelleren Entwicklung der Neurone, so dass bereits innerhalb von 10 Tagen funktionsfähige Zellen erhalten werden.

Des Weiteren wird ein Verfahren zur Förderung der Entwicklung von Neuronen in einem Gewebe beschrieben, welches das Befelden des Gewebes mit einer Hochfrequenzschwingung, die eine Frequenz von 100 bis 200 MHz aufweist, umfasst und dadurch gekennzeichnet ist, dass die Hochfrequenzschwingung durch eine Niederfrequenzschwingung, die eine Frequenz von 5 bis 20 Hz aufweist, moduliert wird. Die Neurogenese findet zu einem ganz überwiegenden Teil während der Entwicklung statt. Im adulten Nervensystem wird hingegen nur noch eine geringe Anzahl von Neuronen gebildet, insbesondere in der Subventrikulärzone und im Hippocampus. Viele Erkrankungen und Verletzungen gehen jedoch mit einem neuronalen Verlust einher, so dass eine Neubildung von Neuronen gewünscht ist. Hierzu zählen beispielsweise neurodegenerative Erkrankungen wie Parkinson und Multiple Sklerose, aber auch Verletzungen des Gehirns oder des Rückenmarks. Interessanterweise verfügt das adulte Nervensystem durchaus über endogene neuronale Vorläufer, die sich jedoch nur verhältnismäßig selten teilen und kaum Neurone bilden. Zudem gestaltet sich die Integration der wenigen neu gebildeten Neurone in die bestehende neuronale Netzwerke als schwierig. In Tierversuchen konnte jedoch gezeigt werden, dass die im adulten Gehirn vorhandenen neuronalen Vorläuferzellen zur Neurogenese stimuliert werden können, beispielsweise durch erhöhte Bewegung und verstärkte geistige Forderung der Tiere. Eine ähnliche Stimulation der Neurogenese konnte *in vitro* durch Befelden neuronaler Vorläuferzellen erreicht werden. Die Befeldung ist für die Stimulation der Neurogenese von körpereigenen adulten neuronalen Vorläuferzellen besonders geeignet, da es sich hier um ein Verfahren ohne invasiven Eingriff handelt. Die Befeldung kann beispielsweise durch Anlegen einer oder mehrerer Befeldungskapseln an die Körperstellen des Patienten, an denen die Entwicklung neuronaler Zellen gefördert werden soll, erfolgen. Bestrahlungs- bzw. Befeldungsvorrichtungen, die hierzu geeignet sind, werden beispielsweise in den folgenden Dokumenten beschrieben: EP 0 136 530 B1, DE 101 00 385 A1, DE 10 2005 014 023 A1 und EP 2 153 869 A1. Durch die Befeldung wird die Bildung neuronaler Zellen durch Vorläuferzellen sowie die Differenzierung und Reifung der gebildeten Neurone gefördert. Das Verfahren ist somit geeignet die körpereigene adulte Neurogenese zu fördern, insbesondere in Gewebe, welche aufgrund einer neurodengerativen Erkrankung geschädigt sind.

Dementsprechend wird ein Verfahren zur Behandlung eines Patienten beschrieben, der an einer Erkrankung des Nervensystems leidet, wobei ein erkrankter Teil des Nervensystems mit einer Hochfrequenzschwingung, die eine Frequenz von 100 bis 200 MHz aufweist, befeldet wird, dadurch gekennzeichnet, dass die Hochfrequenzschwingung durch eine Niederfrequenzschwingung, die eine Frequenz von 5 bis 20 Hz aufweist, moduliert wird. Dieses Verfahren ist insbesondere geeignet um Demenzen, Alzheimer, Apoplexe, Polypathien, Parkinson oder Multipler Sklerose zu behandeln. Dem Patienten kann zusätzlich ein Antidementiva, bevorzugt ein Acetylcholinesterasehemmer, verabreicht werden. Ein geeignete Acetylcholinesterasehemmer ist beispielsweise Donepezil.

Darüber hinaus ist das Verfahren auch zur Behandlung von Verletzungen des Nervensystems, insbesondere des Rückenmarks geeignet. Nach einer Verletzung des Nervensystems werden die endogenen Vorläuferzellen durch körpereigene Mechanismen zur Proliferation angeregt. Dabei kommt es aber überwiegend zur Bildung von Glia Zellen, welche das verletzte Gewebe abschirmen und die sogenannte gliale Narbe (englisch: glial scare) bilden. Die übermäßige Bildung von Glia Zellen verhindert jedoch auch die Bildung und Integration neuer Neurone, die die funktionellen Schäden der Verletzung potentiell ausgleichen könnten. Durch Befeldung des verletzten Gewebes kann die Bildung von Neuronen unter Reduktion der Bildung von Glia Zellen gefördert werden. Zudem fördert die Befeldung die Differenzierung der Neurone, wodurch die Wahrscheinlichkeit der Integration funktioneller neuer Nervenzellen erhöht wird.

### Beispiele

### Material und Methoden

Sofern nichts anderes angegeben ist, wurden alle Experimente unter Verwendung der im Folgenden beschriebenen Materialien und Methoden ausgeführt.

### Zellkultur

Für die Nervenzellkultur wurde Gewebe aus dem frontalen Kortex und dem Hippocampus fötaler Mäuse verwendet. Nach der Präparation des Gewebes wurde dieses enzymatisch und mechanisch dissoziiert. Die Zellsuspension wurde dann auf die mit Poly-D-Lysin und Laminin vorbehandelten Oberflächen der Neurochips ausgesät. Die Kulturen erhielten in Abhängigkeit von der Zelldichte nach 4 bis 5 Tagen eine antimitotische Behandlung zur Eindämmung des übermäßigen Wachstums nichtneuronaler Zellen. Bis zur Messung der elektrischen Aktivität nach 4 - 6 Wochen wurden sie dreimal wöchentlich mit frischem Nährmedium (Dulbecco's Modified Eagles Medium; DMEM) versorgt. Dieses war frei von Antibiotika und enthielt 10 % Pferdeserum, um die Bedingungen für die Zellentwicklung so natürlich wie möglich zu halten.

### Prüfmethode

Da neuronale Netzwerkkulturen *in vitro* spontane Aktivität entwickeln und ausgeprägte Signalmuster zeigen, die hoch sensitiv auf ihre chemische Umgebung reagieren, können sie auch als zuverlässiges Prüfsystem für eine schnelle Bestimmung neuroaktiver und -toxischer Verbindungen und Umgebungszustände genutzt werden. Es kommt zur Kopplung zwischen Elektroden des Chips und den Nervenzellen, wodurch die Aktionspotentiale der Zellen abgeleitet und das elektrische Aktivitätsmuster ausgewertet werden kann. Die direkt auf den Neurochips auswachsenden Zellen entwickeln sich zu natürlichen Nervennetzwerken. Diese sind, dem Ursprungsgewebe vergleichbar, aus einem Gemisch von Neuronen und Glia Zellen zusammengesetzt, wobei die Glia Zellen in Wechselwirkung mit den Neuronen vielfältige Funktionen beim Metabolismus und Stofftransport erfüllen.

Die Zellkulturen zeigen bereits nach einer Woche elektrische Aktivität und nach drei Wochen erste Muster zeitlich begrenzter Folgen von Aktionspotentialen (Aktivitätsphasen). Typischerweise sind die Aktionspotentiale zeitlich in Ruhe- und Aktivitätsphasen, so genannte "Bursts" gruppiert. Diese Bursts sind ein wichtiges Merkmal im Aktivitätsmuster der Netzwerke. Aktionspotentiale werden auch als "spikes" bezeichnet. Die Netzwerke sind auf den Neurochips ein Jahr und länger langzeitstabil.

Für die Messungen wurde ein am Center for Network Neuroscience, University of North Texas, hergestellter Neurochip (Mikroelektrodenassay, MEA) verwendet. Die Neurochips bestehen aus einem 50 x 50 x 1 mm großen, mit Indium-Zinn-Oxid (ITO) beschichteten Quarzglasplättchen, auf welchem die ITO-Leiterbahnen photolithographisch herausgearbeitet wurden, welches dann mit einem Polymer isoliert wurde und auf dem anschließend die Elektroden mit einem Laser freigelegt und elektrolytisch vergoldet wurden. Von den neuronalen Netzwerken können von parallel maximal 256 Neuronen Aktionspotentiale extrazellulär aufgezeichnet werden. Die mit diesen Neurochips routinemäßig aufgenommenen Signale liegen in einem Bereich von 100 - 1000 µV. Die Aufnahme (Abtastrate von 40 kHz) und Verarbeitung der Signale erfolgt mit einem computergesteuerten 64-Kanal-Verstärkersystem (Plexon, Inc., Texas, USA). Nach dem Aufsetzen der MEA-Neurochips nach 4 Wochen div wurde die native Spontanaktivität für mindestens 1Stunde aufgezeichnet.

### Datenanalyse

Die Auswertung der aufgezeichneten Daten basierte auf der Änderung der Netzwerk-Aktivitätsmuster. Die Analyse dieser Aktivitätsmuster erfolgte im NeuroExplorer, (Plexon, Inc., Texas, USA). Die Daten wurden in 60 Sekunden Abschnitte strukturiert, und für chronisch beeinflusste Spontanaktivität (stabile Phase von 30 min) wurden Mittelwerte der Aktivitätsmerkmale berechnet (Absolutwerte). Es wurden 31 primären und statistischen Aktionspotential- und Burst Merkmale berechnet. Die statistischen Merkmale CV_{ZEIT} und CV_{NET} (Koeffizient der Variation = Standardabweichung/Mittelwert*100%, berechnet über die Zeit und über das Netzwerk) geben Aufschluss über Oszillationsverhalten (CV_{ZEIT}) und Synchronität (CV_{NET}) des Aktivitätsmusters für die verschiedenen Experimentepisoden.

Die Auswertung der Aktivität konzentrierte sich auf vier Gruppen: die Beschreibung der allgemeinen Netzwerkaktivität durch die Aktionspotential-Rate (engl.: Aktionspotential rate; SR) und Burst Rate (BR); die Charakterisierung der Burst Struktur durch die Parameter Burst Dauer (engl.: Burst duration, BD), Burst Fläche, Burst Plateau und Burst Form Triangle 3; sowie auf die Bestimmung der Synchronität des Netzwerkes und die Charakterisierung des Oszillationsverhaltens des Netzwerkes.

### Charakterisierung morphologischer Effekte

Nach Aufzeichnung der Netzwerkaktivität wurden die Zellen morphologisch mittels Immunfärbungen und Fluoreszenzmikoskopie untersucht. Diese Analyse ermöglicht die quantitative Erfassung der Zahl von Neuronen und der Zahl der Neuriten. Beide Parameter zeigen den Effekt der Befeldung auf die Netzwerkmorphologie und zelluläre Zusammensetzung.

Hierfür wurden alle gemessenen MEAs mit Paraformaldehyd (PFA) fixiert und immunzytochemisch auf folgende Marker untersucht: Hoechst/Bisbenzimid für Gesamtzellkerne; beta-3-Tubulin/MAP2für Neuronenausläufer; HuC/D für Neuronenzellkörper. Anschließend wurden mikroskopisch fünf repräsentative Felder pro MEA (Elektrodenfeld, Randgebiete) für jeweils mindestens fünf repräsentative MEAs aus mindestens drei unabhängigen Zellpräparationen pro Befeldungsgruppe und Kontrolle über semi-automatische Bildanalysen quantitativ analysiert.

### Experiment 1

### Versuchsaufbau

Es wurden Kulturen des murinen Kortex (und Hippocampus) während der Kultivierung wiederholt niederfrequent bei 16 Hz (Basisfrequenz von 150 MHz, Modulation 50 %, Leistungsstufe 6, entspricht einer magnetischen Feldstärke von 1 pT und einer Leistungsabgabe von ca. 1,85 mW) befeldet. Die auf MEAs wachsenden Kulturen wurden für eine Stunde mit dem Megawave 150 (Mega Wave GmbH) befeldet, wobei sie in einem separat stehenden Inkubator bei 37°C und 5 % CO₂-Zufuhr gehalten wurden und die Befeldungskapsel (Applikator) des Gerätes unter die MEAs gelegt wurde (**Figur 1**). Aufgrund der Anordnung mehrerer MEAs übereinander konnte auch untersucht werden, inwiefern die Entfernung der Kulturen zum Applikator den Effekt der Befeldung beeinflusst. Alle Kulturen wurden in einem maximalen Abstand von 7 cm vom Applikator befeldet.

Die Befeldung wurde zweimal pro Woche zum Zeitpunkt des Mediumwechsels durchgeführt. Nach 28 Tagen in Kultur und acht Befeldungseinheiten wurde die Netzwerkaktivität auf den MEA-Ableitstationen untersucht. Es wurden insgesamt drei Experimentserien durchgeführt: Befeldung/Nichtbefeldung.

### Ergebnisse

Nachdem die kortikalen Netzwerke auf den MEAs von 5 bis 28 Tagen *in vitro* befeldet wurden, wurde die spontane Netzwerkaktivität aufgezeichnet. Anschließend wurde das Netzwerkaktivitätsmuster der Befeldung und der Kontrollgruppe ausgewertet und die Effekte der Befeldung gegen die unbehandelte Kontrolle verglichen. Der Vergleich der durch die beiden Befeldungsgruppen induzierten Netzwerkaktivitätsänderungen erfolgte in vier Kategorien: allgemeine Aktivität, Burst Struktur, Oszillation und Synchronität.

Durch die direkte Befeldung (Gruppe 1) wurde die Burst Struktur verändert. Die Burst Dauer wurde in beiden Befeldungsgruppen signifikant kürzer. Die Anzahl der Aktionspotentiale in Bursts wurde ebenfalls verringert, wohingegen die Frequenz der Aktionspotentiale leicht anstieg (**Figur 2**). In der Befeldungsgruppe 2 wurden ebenfalls Änderungen der Burst Struktur beobachtet, die sich wiederum in einer Verkürzung der Burst Länge verbunden mit einer Reduktion der Aktionspotentiale pro Burst äußerte. Die Erhöhung der Frequenz der Aktionspotentiale im Burst war zwar geringer als bei der Befeldungsgruppe 1, allerdings unterschied sie sich signifikant von der Kontrollgruppe (**Figur 3**). Das Oszillationsverhalten und die Synchronität der Netzwerkaktivität blieb durch die Befeldung unverändert **(****Figur 2** **und** **3**).

Ähnliche Veränderungen wurden auch bei Hippocampuskulturen beobachtet. Insbesondere wurden bei der Befeldungsgruppe 2 Änderungen der Burst Struktur, wie die Verkürzung der Burst Länge und eine Reduktion der Aktionspotentiale pro Burst, beobachtet. Auch in diesen Kulturen blieb das Oszillationsverhalten und die Synchronität der Netzwerkaktivität unverändert.

### Zusammenfassung

Die Befeldung induzierte in beiden Befeldungsgruppen Änderungen in der Burst Struktur, die sich in einer Verkürzung der Burst Länge verbunden mit einer Reduktion der Aktionspotentiale pro Burst äußerte. Somit ähneln die durch die Befeldung induzierten Änderungen den aktivitätssteigernden Effekten bekannter prokognitiver Substanzen, wie beispielsweise Donepezil.

### Experiment 2

### Versuchsaufbau

Es wurden Kulturen des murinen Kortex (und Hippocampus) während der Kultivierung wiederholt niederfrequent bei 16 Hz (Basisfrequenz von 150 MHz, Modulation 50 %, Leistungsstufe 3, entspricht einer magnetischen Feldstärke von 0,125 pT und einer Leistungsabgabe von ca. 0,12 mW) befeldet. Die auf MEAs wachsenden Kulturen wurden für eine Stunde mit dem Megawave 150 (Mega Wave GmbH) befeldet, wobei sie in einem separat stehenden Inkubator bei 37°C und 5 % CO₂-Zufuhr gehalten wurden und die Befeldungskapsel (Applikator) des Gerätes unter die MEAs gelegt wurde (**Figur 1**). Aufgrund der Anordnung mehrerer MEAs übereinander konnte auch untersucht werden, inwiefern die Entfernung der Kulturen zum Applikator den Effekt der Befeldung beeinflusst. Die Abstände der Befeldungsgruppen von der Sonde betrugen hierbei: 1a=2 mm, 1b=18 mm, 2a=34 mm; 2b=50 mm. Alle Kulturen wurden in einem maximalen Abstand von 7 cm vom Applikator befeldet.

Die Befeldung erfolgte zwei Mal pro Woche zum Zeitpunkt des Mediumwechsels im Zeitraum von 5 - 28 Tagen in Kultur. Anschließend wurde die spontane Netzwerkaktivität aufgezeichnet. Das Netzwerkaktivitätsmuster der Befeldungsgruppen und der Kontrollgruppe wurde ausgewertet und die Effekte der Befeldung gegen die unbehandelte Kontrolle verglichen. Der Vergleich der durch die beiden Befeldungsgruppen induzierten Netzwerkaktivitätsänderungen erfolgt in 4 Kategorien: allgemeine Aktivität, Burst Struktur, Oszillation und Synchronität.

Nach der Analyse der Netzwerkaktivität wurden die gemessenen MEAs fixiert und morphologisch mittels Immunfärbungen und Fluoreszenzmikroskopie untersucht. Dabei wurden die folgenden morphologischen Parameter berücksichtigt: Gesamtzellzahl, Anteil an Neuronen und Menge an Neuriten, sowohl absolut als auch relativ zur Neuronenzahl.

### Ergebnisse

Die direkte, effektive chronische Befeldung (Gruppe 1a) kortikaler Netzwerke während der Ausdifferenzierung (5-28 div) führte im Trend zu einer verringerten Allgemeinaktivität. Dieses spiegelt sich in der verringerten Aktionspotential Rate, einer Reduzierung der Burst Struktur (Verkürzung der Burst Länge und Verkleinerung der Burst Fläche) und einer Abnahme der Synchronität. Die 34 - 50 mm vom Applikator entfernte, schwächere Befeldungsgruppe 2 induzierte eine Stärkung der Burst Struktur, was sich in einer Verlängerung der Burst Länge und Vergrößerung der Burst Fläche zeigt (**Figur 5**). Unter den gegebenen Versuchsbedingungen zeigt sich ein Trend, dass mit zunehmendem Abstand von der Befeldungssonde (**Figur 6**) sich die Burst Struktur verstärkt, die Aktionspotential- und Burst Rate aber unverändert bleiben (**Figur 6**). Diese Ergebnisse sind im Einklang mit den durch die Befeldung induzierten morphologischen Veränderungen.

Die Befeldung mit Stärke 3 induzierte eindeutige vom Abstand abhängige (Befeldungsgruppe 1 vs. 2) morphologische Veränderungen in den kortikalen Netzwerken. Mit zunehmendem Abstand von der Befeldungssonde nimmt der prozentuale Anteil der Neurone zu (**Figur 7**). Hierbei bleibt die Gesamtzahl der Zellen unverändert, bzw. nimmt leicht ab (**Figur 7**). Es kommt offensichtlich zu einer Verschiebung der Vorläuferzellpopulationen in Richtung Neuronen gegenüber Glia Zellen. Im Gegensatz zur Neuronenzahl nimmt mit steigendem Abstand die Anzahl der Neurite pro Neuron ab (**Figur 7**). Dies deutet darauf hin, dass es durch die Befeldung zur verstärkten Entwicklung bipolarer Interneurone kommt.

Die Befeldung mit Stärke 3 führte im Vergleich zu Stärke 6 (siehe Experiment 1) in beiden Abstandsgruppen (Befeldungsgruppe 1: **Figur 8**; Befeldungsgruppe 2: **Figur 9**) zu einem relativen Aktivitätsanstieg. Dieser zeigte sich in einem Anstieg der Aktionspotential- und Burst Rate, einer verstärkten Burst Struktur und einer erhöhten Synchronität.

### Zusammenfassung

Im Experiment 2 zeigte die Befeldung mit Stärke 3 im Vergleich zu Stärke 6 (Experiment 1) in beiden Abstandsgruppen (Befeldungsgruppe 1: **Figur 8**; Befeldungsgruppe 2: **Figur 9**) einen Aktivitätsanstieg. Ferner wurde der Trend beobachtet, dass sich mit zunehmendem Abstand von der Befeldungssonde die Burst Struktur verstärkt, wobei die Aktionspotential- und Burst Rate unverändert bleiben. Diese Ergebnisse wurden durch die morphologischen Daten bestätigt. Hier zeigte sich, dass die Befeldung mit Stärke 3 eindeutige vom Abstand abhängige morphologische Veränderungen in den kortikalen Netzwerken induzierte. Mit zunehmendem Abstand von der Befeldungssonde nimmt der Anteil an Neurone, insbesondere an Interneuronen zu.

### Experiment 3

### Versuchsaufbau

Es wurden Kulturen des murinen Kortex (und Hippocampus) während der Kultivierung wiederholt niederfrequent bei 10 Hz und alterierend 10 und 16 Hz (Basisfrequenz von 150 MHz, Modulation 50 %, Leistungsstufe 3, entspricht einer magnetischen Feldstärke von 0,125 pT und einer Leistungsabgabe von ca. 0,12 mW) befeldet. Die auf 12-well MEAs wachsenden Kulturen wurden für eine Stunde mit dem Megawave 150 (Mega Wave GmbH) befeldet, wobei sie in einem separat stehenden Inkubator bei 37°C und 5 % CO₂-Zufuhr gehalten wurden und die Befeldungskapsel (Applikator) des Gerätes unter die MEAs gelegt wurde (**Figur 1**). Die alternierende Frequenz wurde je Befeldungseinheit 30 min bei 10 Hz und anschließend 30 min bei 16 Hz durchgeführt. Aufgrund der Anordnung mehrerer MEAs übereinander konnte auch untersucht werden, inwiefern die Entfernung der Kulturen zum Applikator den Effekt der Befeldung beeinflusst. Die Abstände zur Oberfläche der Kapsel betrugen 41 mm (Abstand A2) und 83 mm (Abstand A3). Die Kulturen wurden über den technischen Maximalabstand von 7 cm Abstand zum Applikator hinaus befeldet um die Abstandseffekte über diese Grenze hinaus zu untersuchen.

Das 12-well MEA bietet die Möglichkeit, die Netzwerke während der Kultivierung und Befeldung mehrmals durch elektrische Ableitung zu untersuchen. Somit konnten einerseits die Befeldungseffekte während der neuronalen Entwicklung nach 14, 21 und 28 Tagen in Kultur (4, 6 bzw. 8 Befeldungseinheiten) auf den MEA - Ableitstationen untersucht werden und Aufschluss über eine minimale Befeldungsdauer geben. Andererseits war es durch die zusätzlichen Datenpunkte und eine erhöhte Anzahl an Einzelexperimenten je Gruppe möglich, die Effektgrößen zu vergleichen und die am stärksten signifikante Parameterkonstellation zu isolieren.

Die multiparametrische Auswertung der Netzwerkparameter gab Aufschluss darüber, wie sich die Netzwerkaktivität und somit die funktionelle Verbindung der Neurone untereinander im Vergleich zur Kontrolle und zum Vorzeitpunkt (14, bzw. 21 Tage) verändert hat. Funktionelle Parameter, welche die Grundaktivität, die Burst Struktur, die Netzwerkoszillation oder die zeitliche Synchronisation beschreiben, gaben Aufschluss darüber wie sich die funktionelle Netzwerkarchitektur durch die Befeldung ändert.

Nach der letzten Messung am Tag 28 wurden die Zellen morphologisch mittels Immunfärbungen und Fluoreszenzmikroskopie untersucht. Diese Analyse ermöglichte die quantitative Erfassung der Zahl von Neuronen. Hierfür wurden alle gemessenen MEA mit PFA fixiert und immunzytochemisch für die Gesamtzellkerne, die Neurone mit HuCD und die GABAergen Neurone mit anti-GABA Antikörpern gefärbt. Anschließend wurden mikroskopisch drei repräsentative Felder pro 12-well MEA für jeweils drei repräsentative MEAs pro Befeldungsgruppe und Kontrolle quantitativ analysiert. Es wurden die Parameter Gesamtzellzahl, % Neuronenanteil, Anzahl GABAerger Neurone absolut und relativ zur Neuronenzahl ermittelt.

### Ergebnisse

Die chronische Befeldung unter den verschiedenen Bedingungen zeigte zeitabhängige Effekte auf die allgemeine Qualität der Differenzierung. Die Qualität (=Messbarkeit) der Netzwerke in der unbehandelten Kontrollgruppe war mit 79 %, 67 % bzw. 70 % MEAs mit aktiven Neuronen nach 14, 21 bzw. 28 relativ hoch und entsprach den statistischen Erwartungen.

### Effekte nach 10-tägiger Befeldungsdauer am Tag 14 div

Befeldungsgruppe 10+16 Hz, A3: Die direkte, effektive chronische Befeldung der kortikalen Netzwerke mit alternierender Frequenz von 10 und 16 Hz und einem Abstand von 83 mm für 10 Tage führte zu einer Erhöhung der allgemeinen Aktivität (**Figur 10**; Aktionspotential Rate ↑, Burst Rate ↑). Die Parameter dieser Befeldungsgruppe induzierten ferner eine Verlängerung der Dauer der Bursts um 36 % von 250 auf 340 ms.

Befeldungsgruppe 10+16 Hz, A2: Im Abstand von 41 mm induzierte die chronische Befeldung mit alterierend 10/16 Hz keine signifikanten Aktivitätsänderungen.

Im Trend ist die Burst Struktur jedoch verstärkt (Burst Plateau ↑, Burst Amplitude ↑, Aktionspotentialdichte ↑).

### Effekte nach 17-tägiger Befeldungsdauer am Tag 21 div

Befeldungsgruppe 10+16 Hz, A3: Die direkte, effektive chronische Befeldung der kortikalen Netzwerke mit alternierender Frequenz von 10 und 16 Hz und einem Abstand von 83 mm führte zu einer Erhöhung der allgemeinen Aktivität in Aktionspotential- und Burst Rate um jeweils 21 und 12 % (**Figur 11**). Es wurde keine Veränderung in der Burst Struktur beobachtet. Die Synchronität nahm zu, wohingegen die Regelmäßigkeit des Aktivitätsmusters unverändert blieb.

Befeldungsgruppe 10+16 Hz, A2: Im Abstand von 41 mm induzierte die chronische Befeldung mit alterierend 10/16 Hz keine Änderungen der allgemeinen Aktivität. Die Parameter dieser Befeldungsgruppe induzierten jedoch einen Anstieg der Burst Dauer um 11 % von 236 auf 370 ms.

### Effekte nach 24-tägiger Befeldungsdauer am Tag 28 div

Befetdungsgruppe 10+16 Hz, A3: Die direkte, effektive chronische Befeldung der kortikaler Netzwerke mit alternierender Frequenz von 10 und 16 Hz und einem Abstand von 83 mm führte zu einer Erhöhung der allgemeinen Aktivität in Aktionspotential - und Burst Rate um jeweils 10 % **(****Figur 12**). Die Befeldung induzierte ferner eine Verstärkung der Burst Struktur, was sich in einer Vergrößerung der Burst Plateau, der Burst Amplitude und einer Erhöhung der Anzahl an Aktionspotentialen im Burst zeigte. Die Synchronität und die Regelmäßigkeit des Aktivitätsmusters blieben unverändert.

Befeldungsgruppe 10+16 Hz, A2: Im Abstand von 41 mm induzierte die chronische Befeldung mit 10/16 Hz keine Änderungen in der allgemeinen Aktivität, die Burst Struktur wurde jedoch verstärkt (Vergrößerung des Burst Plateau, der Burst Amplitude und einer Erhöhung Anzahl an Aktionspotentialen im Burst; **Figur 12**).

### Zeitabhängige Effekte der chronischen Befeldungsvariationen

Die Befeldung mit alternierenden 10 und 16 Hz induzierte eine Beschleunigung der allgemeinen Aktivitätsentwicklung (**Figur 13**). Nach 10-tägiger Befeldung (14 div) hatte die allgemeine Aktivität in ihrer Aktionspotential Rate das Niveau eines kortikalen Netzwerkes nach 28 div erreicht. Nach 17-tägiger Befeldung (21 div) wurde auch die Burst Rate eines kortikalen Netzwerkes nach 28 div erreicht.

### Effekte der Befeldungsmodulationen auf die Morphologie

Befeldungsgruppe 10+16 Hz, A3: Die Befeldung mit alternierenden Frequenzen von 10 und 16 Hz erzeugte einen Anstieg der absoluten Neuronenzahl um 19 % (**Figur 15**) und des prozentualen Anteils der Neurone um 22 % (**Figur 15**). Innerhalb der neuronalen Population stieg der Anteil der GABAergen Neurone um 24 % (**Figur 15**).

Befeldungsgruppe 10+16 Hz, A2: Die Befeldung mit alternierenden Frequenzen im Abstand A2 induzierte bei gleicher Neuronenzahl und gleichem GABAergen Anteil keine morphologischen Änderungen in den kortikalen Netzwerken (**Figur 15**).

Befeldungsgruppe 10 Hz, A3: Durch die Befeldung mit einer Frequenz von 10 Hz im Abstand A3 wurde der Anteil GABAerger Neuronen mit 16 % leicht erhöht.

Befeldungsgruppe 10 Hz, A2: Die Befeldung mit einer Frequenz von 10 Hz erzeugte einen Anstieg der absoluten Neuronenzahl um 12 %. Ebenso nahm der prozentuale Anteil der Neurone um 28 % zu. Innerhalb der neuronalen Population stieg der Anteil der GABAergen Neurone um 25 % (**Figur 15**).

### Zusammenfassung

Befetdungsgruppe 10+16 Hz, A2: Im Abstand A2 induzierte die chronische Befeldung mit 10/16 Hz eine Vergrößerung der Burst Struktur, während die allgemeine Aktivität unverändert blieb.

Befeldungsgruppe 10+16 Hz, A3: Die Befeldung mit alternierenden 10 und 16 Hz im Abstand A3 induzierte eine Beschleunigung der allgemeinen Aktivitätsentwicklung. Des Weiteren erzeugte sie einen signifikanten Aktivitätsanstieg und eine Vergrößerung der Burst Struktur. Diese Aktivitätsänderungen waren verbunden mit einem Anstieg der Gesamtneuronenzahl und dem Anstieg des Anteils GABAerger Neurone.

Befeldungsgruppe 10 Hz, A2: Im Abstand A2 erzeugte die chronische Befeldung mit 10 Hz keine Änderungen der kortikalen Netzwerkaktivität. Es wurde jedoch ein Anstieg der Neuronenzahl und des Anteils GABAerger Neurone ermittelt.

Zusammengefasst zeigt Experiment 3, dass mit größerem Abstand A3 von der Befeldungskapsel bei einer Befeldung mit alternierenden Frequenzen von 10 und 16 Hz die allgemeine Aktivität erhöht wird und sich die Burst Struktur verstärkt. Dieser Effekt war nach 10-tägiger Befeldung, das heißt nach 14 div, am deutlichsten ausgeprägt. Insgesamt wurde die Entwicklung der kortikalen Aktivität unter diesen Bedingungen also beschleunigt. Diese Ergebnisse wurden durch die morphologischen Daten bestätigt, die zeigen, dass bei unveränderter Gesamtzellzahl der prozentuale Anteil der Neurone zu nahm.

Die durch die Befeldung induzierten Effekte auf die sich entwickelnde Netzwerkaktivität ähneln den aktivitätssteigernden Effekten, die bei Zugebe von Donepezil oder dem Wachstumsfaktor BNDF beobachtet werden.

**Tabelle 1: Einfluss der verschiedenen Befeldungsvariationen auf die Netzwerkaktivität und Morphologie kortikaler Zellkulturen.**

| **Befeldung** | **Netzwerkaktivität** | | | | **Morphologie** | |
|---|---|---|---|---|---|---|
| | allgem. Aktivität | Burst Struktur | Synchronität | Oszillation | Neurogenese | GABAerge Neurone |
| ctrl | | | | | | |
| 10Hz,A2 | - | - | - | - | ↑ | ↑ |
| 10+16Hz,A2 | - | ↑ | - | - | - | - |
| 10Hz,A3 | ↓ | ↓ | ↑ | ↑ | - | - |
| 10+16Hz,A3 | ↑ | ↑ | - | - | ↑ | ↑ |

### Experiment 4

### Versuchsaufbau

Kulturen des murinen Kortex wurden kultiviert und alterierend mit 10 bzw 16 Hz befeldet, wie in Experiment 3 beschrieben. Der Abstand der Befeldungsspule zu den Zellen betrugt 83 mm. Ein Teil der Zellen wurde parallel zur Befeldung mit jedem Mediumwechsel mit 300 nM Donepezil (Arizept®) behandelt. Es wurden insgesamt vier Gruppen an 2 Messzeitpunkten 14 und 28 *Tage in vitro* untersucht: (1) Vehicle-Kontrolle, (2) Donepezil (300 nM)-Behandlung, (3) Vehicle-Kontrolle 10/16 Hz Befeldung (10+16 Hz, A3), und (4) Donepezil-Behandlung (300 nM) + 10/16 Hz Befeldung (10+16 Hz, A3 + Donepezil).

Die multiparametrische Auswertung der Netzwerkparameter gab Aufschluss darüber, wie sich die Netzwerkaktivität durch Donepezil allein und in Kombination mit der Befeldung an jedem Messzeitpunkt und im Vergleich der beiden Zeitpunkte (14 und 28 Tage) verändert. Funktionelle Parameter, welche die Grundaktivität, die Burst Struktur, die Netzwerkoszillation oder die zeitliche Synchronisation beschreiben, gaben Aufschluss darüber wie sich die funktionelle Netzwerkarchitektur durch die Befeldung ändert.

Durch die Ergebnisse der Experimente 1 bis 3 konnten deutliche Effekte auf die Neuronenpopulation gezeigt werden. Ferner ist bekannt das Donepezil einen stimulierenden Einfluss auf die Neuriten und Synapsendichte im HippocampusKulturen hat. Um die (zusätzlichen) Effekte der Befeldung auf die Synaptogenese und/oder Neurogenese zu klären, wurden neben der Neuronenpopulation und der GABAergen Subpopulation auch diese beiden Parameter erhoben. Die Experimente 1 bis 3 haben auch gezeigt, dass der funktionelle Effekt nach 14 Tagen *in vitro* (14 DIV) am stärksten war, was auf eine Beschleunigung der neuronalen Differenzierung hinweist. Neben der Endmessung an Tag 28 wurden daher auch an Tag 14 *in vitro* morphologische Analysen durchgeführt. Hierfür wurden Zellen auf Glasdeckgläschen in 12 Well Platten ausgesät und analog zu den Multiwell-MEAs behandelt und befeldet, wie in Experiment 3 beschrieben. Diese Zellen wurden an Tag 14 bzw. Tag 28 *in vitro* fixiert, immunzytochemisch (Neuriten, Neuronen, Zellkerne, Synapsen, GABAerge Neurone) markiert und semi-quantitativ morphologisch analysiert.

### Ergebnisse

Die nachfolgenden Ergebnisse wurden aus insgesamt 4 biologischen Wiederholungen gewonnen. Die Stichprobenzahl ist in Tabelle 2 angegeben:

**Tabelle 2:**

| Elektrophysiologische Messungen | **Ctrl** | **DPZ** | **10+16 Hz** | **10+16Hz + DPZ** |
|---|---|---|---|---|
| **14DIV MEA record.** | n=17 | n=22 | n=10 | n=8 |
| **28DIV MEA record.** | n=15 | n=12 | n=9 | n=12 |

| Immunhistochemische Färbungen | **Ctrl** | **DPZ** | **10/16 Hz** | **10/16 Hz+DPZ** |
|---|---|---|---|---|
| **14DIV GABA** | 11 | 10 | 14 | 10 |
| **14DIV Syn** | 12 | 10 | 11 | 11 |
| **28DIV GABA** | 10 | 8 | 10 | 9 |
| **28DIV Syn** | 8 | 8 | 8 | 8 |

| | | | | |
|---|---|---|---|---|
| n= Anzahl der untersuchten Netzwerke | | | | |

### Effekte nach 10-tägiger Befeldungsdauer und Donepezil-Behandlung an Tag 14 in vitro

Donepezil: Nach 10-tägiger wiederholter Behandlung induzierte Donepezil nur marginale Effekte in der Aktivitätsänderung. Die allgemeine Aktivität blieb unverändert. Lediglich eine geringe Verstärkung der Burst Struktur war zu erkennen, die sich in einem Anstieg der Burst Dauer, Burst Fläche und Burst Gestalt (engl.: burst shape triangle 2) um jeweils 16, 8, und 16%, äußerte. Die Regularität der Bursts und die Synchronität der Netzwerke blieb hingegen unverändert (**Figur 16**).

Befeldunq 10+16 Hz, A3: Die Befeldung der kortikalen Netzwerke mit alternierender Frequenz von 10 und 16 Hz und einem Abstand A3 von 83 mm für 10 Tage führte zu einer Erhöhung der allgemeinen Aktivität, was sich in einem nicht signifikanten Anstieg der Spike Rate, Burst Rate und des Spike Kontrastes um jeweils 29, 19 und 47 % zeigte. Auch hier zeigte sich eine Verstärkung der Burst Struktur, die sich im Trend in einem Anstieg der Burst Dauer, Burst Fläche und Burst Gestalt um jeweils 11, 21, und 38 %, äußerte. Das Oszillations- und das Synchronitätsverhalten der Netzwerkaktivität nahmen hingegen ab (**Figur 16**).

Befeldung 10+16 Hz. A3 + Donepezil: Die Befeldung der kortikalen Netzwerke mit zusätzlicher Gabe von Donepezil für 10 Tage führte zu einer Erhöhung der allgemeinen Aktivität, wie ein signifikanter Anstieg der Spike Rate zeigte. Auch hier war eine Verstärkung der Burst Struktur zu beobachten, die sich in einem Anstieg der Burst Dauer, Burst Fläche und Burst Amplitude um jeweils 47, 34, und 95 %, äußerte. Das Synchronitätsverhalten der Netzwerkaktivität und die regelmäßige Struktur der Burst Gestalt nahm ab, wohingegen das Netzwerkoszillationsverhalten unverändert blieb (**Figur 16**).

### Effekte nach 24-tägiger Befeldungsdauer und Donepezil-Behandlung an Tag 28 in vitro

Donepezil: Nach 24-tägiger Behandlung induzierte Donepezil einen nichtsignifikanten Anstieg in der allgemeinen Aktivität (Anstieg in der Spike Rate um 21 %). Die Burst Struktur wurde verstärkt, was sich in einem Anstieg der Burst Dauer, Burst Fläche, Burst Amplitude und Burst Gestalt um jeweils 75, 35, 21 und 130 %, äußerte. Die Regelmäßigkeit der Bursts (Oszillationsverhalten) blieb unverändert, wohingegen die Netzwerksynchronität anstieg (**Figur 17**).

Befeldunq 10+16 Hz, A3: Die Befeldung der kortikalen Netzwerke führte zu einer Erhöhung der allgemeinen Aktivität mit einem nicht signifikanten Anstieg der Spike Rate, Burst Rate und des Spike Kontrastes um jeweils 43, 29 und 23 %. Gegenüber den Effekten an Tag 14 DIV sind die Effekte auf der Burst Struktur zurückgegangen, es ist jedoch noch ein Anstieg der Burst Fläche um 89 %, zu verzeichnen. Das Oszillationsverhalten und das Synchronitätsverhalten blieben unverändert (**Figur 17**).

Befeldung 10+16 Hz, A3 + Donepezil: Bei der Befeldung der kortikalen Netzwerke mit zusätzlicher Gabe von Donepezil war die allgemeine Aktivität an Tag 28 *in vitro* unverändert. Die Spike Rate zeigte jedoch einen Abfall um 20% und die Burst Dauer verkürzte sich. Das Oszillationsverhalten blieb unverändert, wohingegen das Synchronitätsverhalten numerisch zunahm (**Figur 17**).

### Effekte nach 10-tägiger Befeldungsdauer und Donepezil-Behandlung auf die Morphologie von Frontalkortex-Netzwerken an Tag 14 in vitro

Donepezil: Die wiederholte Behandlung mit 300 nM Donepezil induzierte im Trend einen Anstieg der absoluten und prozentualen Neuronenzahl um jeweils 49 und 54 % (Gesamtzellzahl unverändert). Die Anzahl der Neurite pro Neuron nahmen um 11% ab (nicht signifikant). Der Anteil der GABAergen Neurone innerhalb der neuronalen Population sank um 31 %, wohingegen die Anzahl der Synapsen pro Neurit blieb unverändert (**Figur 18**).

Befeldung 10+16 Hz, A3: Die Befeldung führte zu einem leichten Anstieg der absoluten Neuronenzahl um 19% (Gesamtzellzahl unverändert). Dies entspricht einem Anstieg im prozentualen Anteil der Neuronen-Population um 27 %. Die Anzahl der Neurite pro Neuron nahmen im Trend um 11 %, der Anteil der GABAergen Neurone innerhalb der neuronalen Population um 13 % ab. Die Anzahl der Synapsen pro Neurit blieb unverändert (**Figur 18**).

Befeldung 10+16 Hz, A3 + Donepezil: Die gleichzeitige Behandlung mit Donepezil und Befeldung erzeugte einen Anstieg der absoluten Neuronenzahl um 13 % (Gesamtzellzahl unverändert). Dies entspricht einem Anstieg im prozentualen Anteil der Neuronen-Population um 27 %. Die Anzahl der Neurite pro Neuron nahmen um 9%, und der Anteil der GABAergen Neurone innerhalb der neuronalen Population um 19 % ab. Die Anzahl der Synapsen pro Neuron blieb unverändert (**Figur 18**).

### Effekte nach 24-tägiger Befeldungsdauer und Donepezil-Behandlung auf die Morphologie von Frontalkortex-Netzwerken an Tag 28 in vitro

Donepezil: Die Behandlung mit 300 nM Donepezil induzierte keinen Anstieg der absoluten und prozentualen Neuronenzahl. Die Gesamtzellzahl stieg im Trend um 15 %. Die Anzahl der Neurite und Synapsen pro Neuron blieben unverändert. Der Anteil der GABAergen Neurone erhöhte sich um 17% (**Figur 19**).

Befeldung 10+16 Hz, A3: Die Befeldung erzeugte im Trend einen Anstieg der absoluten Neuronenzahl um 24 % und der Gesamtzellzahl um 15 %. Dies entspricht einem Anstieg im prozentualen Anteil der Neuronen-Population um 7 %. Die Anzahl der Neurite pro Neuron nahmen um 9 % ab. Die Anzahl der Synapsen pro Neuron blieb unverändert. Der Anteil der GABAergen Neurone innerhalb der neuronalen Population stieg um 22% (**Figur 19**).

Befeldung 10+16 Hz, A3 + Donepezil: Die gleichzeitige Behandlung mit Donepezil und Befeldung erzeugte nach 24-tägiger Behandlung einen Anstieg der absoluten Neuronenzahl um 42 %, wobei die Gesamtzellzahl um 28 % stieg. Dies entspricht einem Anstieg im prozentualen Anteil der Neuronen-Population um 13 %. Die Anzahl der Neurite pro Neuron nahmen um 15 % ab, wohingegen die Anzahl der Synapsen pro Neuron und der Anteil der GABAergen Neurone unverändert blieb (**Figur 19**).

### Zusammenfassung

Donepezil: Donepezil induzierte in kortikalen Netzwerken einen Anstieg in der allgemeinen Aktivität, verbunden mit einer Verstärkung der Bursts. Dieser Effekt nahm mit der Behandlungsdauer zu. Die Aktivitätsänderungen waren verbunden mit einem Anstieg des Anteils Neurone und des Anteils GABAerger Neurone und einem Abfall der Neuriten pro Neuron. Insgesamt waren die durch Donepezil induzierten Aktivitäts-verstärkenden Effekte nach 24-tägiger Behandlung (28 DIV) deutlicher als an Tag 14 *in vitro*.

10+16 Hz, A3: Die Befeldung mit alternierenden 10 und 16 Hz im Abstand von 83 mm induzierte eine Beschleunigung der allgemeinen Aktivitätsentwicklung. Diese Aktivitätsänderungen waren verbunden mit einem Anstieg des Anteils Neurone und des Anteils GABAerger Neurone und einem Abfall der Neuriten pro Neuron. Insgesamt erhöht die Befeldung mit alternierenden Frequenzen die allgemeine Aktivität und verstärkt die Burst Struktur.

Donepezil 10+16 Hz, A3: Nach 10-tägiger Behandlung ist ein zusätzlicher Effekt im Vergleich zu einer Behandlung mit Donepezil bzw. der Befeldung allein zu beobachten. Dieser zeigt sich in einem Anstieg der allgemeinen Aktivität und einer Modulierung des Burst Musters (Abfall der Burst Rate und des Burst Kontrastes, Anstieg der Burst Dauer und Fläche). Dieser Effekt war begleitet von einem Anstieg in dem prozentualen Neuronenanteil an 14 *in vitro*. Die Gesamtzellzahl als auch der Neuronenanteil blieben auch nach 28 Tagen *in vitro* erhöht.

Insgesamt wurde durch die kombinierte Behandlung mit Donepezil und Befeldung sowohl funktionale als auch morphologische Änderungen hervorgerufen, die vor allem in der frühen Phase der Ausdifferenzierung deutlich über die Effekte von Donepezil bzw Befeldung alleine hinausgehen.

### Experiment 5

### Zellkultur

Dissoziierte kortikale Zellen (*mus musculus,* E15) wurden in DMEM10/10 resuspendiert (1.0 x 10⁶ cells/ml) und 300µl auf Poly-D-Lysine und Laminin vorbehandelten MEA-neurochips (Axion Biosystems Inc, USA) ausgesät. Die Kulturen wurden bei 37°C und 10 % CO₂ für vier Wochen inkubiert, wobei das Kulturmedium zweimal pro Woche gewechselt wurde (DMEM inkl. 10 % Pferdeserum). Um eine Überwucherung der Kultur mit glialen Zellen zu vermeiden, wurden die Kulturen am fünften Tag in Kultur (5 div) mit 5-fluoro-2'-deoxyuridine (25 µM) und uridine (63 µM) behandelt.

### Versuchsaufbau

Die Kulturen wurden von 4 div bis 28 div zweimal die Woche befeldet mit einer 150 MHz Trägerfrequenz, die alterierend mit 10 und 16 Hz moduliert war, befeldet (100 bzw. 62,5 ms Impulse). Pro Befeldungseinheit wurde die Frequenz 30 min bei 10 Hz und anschließend 30 min bei 16 Hz moduliert. Zur Befeldung wurde ein MegaWave MW150 Radiator mit einem Hochfrequenz Oszillatorkreis, der eine Trägerfrequenz von 150 MHz generiert, und einem Niederfrequenz Oszillatorkreis, der eine regulierbare Modulationsfrequenz generiert, verwendet (Modulation 50 %, Leistungsstufe 3) (**Figur 22**). Während der Pulse, betrug die magnetische Induktion auf Grund der modulierten Trägerfrequenz von 150 MHz zwischen 0,06 und 0,12 pT bei einer linearen Anhängigkeit von der Frequenz. Der Abstand der Kulturen von der Antenne des Applikators betrug 83 mm.

### Ergebnisse

### Veränderung der kortikalen Netzwerkaktivität

Nach vier Behandlungen über 10 Tage (14 div) hatte die Befeldung zu einer beschleunigten Entwicklung der Netzwerkaktivität geführt (**Figur 20**). Allgemeine Aktivitätsparameter wie die Rate der Aktionspotentiale, die Rate der Bursts und der Kontrast der Aktionspotentiale waren erhöht (um 155 %, 122 % bzw. 133 %). Ebenso waren die Burst Strukturen verändert, insbesondere war die Burst Länge auf 130 % im Vergleich zur Kontrolle erhöht. Die Synchronität blieb hingegen unverändert.

Nach acht Behandlungen über die Zeit von 24 Tagen (28 div) wurden ähnliche Entwicklungen beobachtet. Die allgemeine Aktivität war erhöht (Rate der Aktionspotentiale: 136 %, Rate der Bursts: 123 %, Kontrast der Aktionspotentiale: 116%). Darüber hinaus war die Burst Länge auf 81 % reduziert, die Burst Amplitude und die Dichte hingegen auf 129 % bzw. 137 % erhöht. Außerdem war zu diesem Zeitpunkt auch die Sychronität verstärkt.

### Veränderung der kortikalen Netzwerkmorphologie

Nach 14 div zeigten die befeldeten Zellen eine beschleunigte Differenzierung in Neurone, die sich in einem Anstieg des Anteils neuronaler Zellen in der Kultur auf 110 % und in einer verstärkten Bildung von Neuriten (Anstieg auf 113 %) zeigte (**Figur 21**). Färbungen für GABAerge Neurone zeigten eine Vergrößerung dieser spezifischen neuronalen Population (auf 119 %). Die absolute Anzahl an Synapsen bleib hingegen unverändert, so dass die Anzahl der Synapsen pro Neurit verringert war.

### Zusammenfassung

Die Befeldung primärer kortikaler Netzwerke *in vitro* mit einer 10/16 Hz modulierten Trägerfrequenz von 150 MHz förderte die allgemeine Netzwerkaktivität und die neuronale Differenzierung. Dieser Effekt war nach 14 div (d.h. nach 10 Tagen Befeldung) am ausgeprägtesten. Die elektrophysiologischen Veränderungen wurden durch entsprechende morphologische begleitet, die eine allgemeine Verstärkung der Neurogenese erkennen lassen. Darüber hinaus wurde durch die Befeldung vor allem die Bildung GABAerger Neurone gefördert.

### Referenzliste

EP 0 136 530 B1
DE 101 00 385 A1
DE 10 2005 014 023 A1
EP 2 153 869 A1.

## Patentansprüche

1. *In vitro* Verfahren zur Förderung der Entwicklung von Neuronen, umfassend die Schritte
- Kultivieren der Neurone aus neuronalen Vorläuferzellen, und
- Befelden der neuronalen Vorläuferzellen und/oder der Neurone während des Kultivierens mit einer Hochfrequenzschwingung, die eine Frequenz von 100 bis 200 MHz aufweist,
**dadurch gekennzeichnet, dass** die Hochfrequenzschwingung durch eine Niederfrequenzschwingung, die eine Frequenz von 5 bis 20 Hz aufweist, moduliert wird.

2. *In vitro* Verfahren nach Anspruch 1, **dadurch gekennzeichnet, dass** die Hochfrequenzschwingung eine Frequenz von 130 bis 170 MHz, ferner bevorzugt von 150 MHz aufweist.

3. *In vitro* Verfahren nach Anspruch 1 oder 2, **dadurch gekennzeichnet, dass** die Niederfrequenzschwingung eine Frequenz von 10 bis 20 Hz, bevorzugt von 10 Hz oder 16 Hz aufweist.

4. *In vitro* Verfahren nach einem der Ansprüche 1 bis 3, **dadurch gekennzeichnet, dass** die Hochfrequenzschwingung abwechselnd durch eine Niederfrequenzschwingung mit einer Frequenz von 10 Hz und 16 Hz moduliert wird.

5. *In vitro* Verfahren nach einem der Ansprüche 1 bis 4, **dadurch gekennzeichnet, dass** die neuronalen Vorläuferzellen und/oder die Neurone durch eine Bestrahlungsvorrichtung befeldet werden und in einem Abstand von 1 bis zu 100 mm, bevorzugt 30 bis 100 mm, ferner bevorzugt 50 bis 100 mm von der Bestrahlungsvorrichtung angeordnet sind.

6. *In vitro* Verfahren nach einem der Ansprüche 1 bis 4, **dadurch gekennzeichnet, dass** die neuronalen Vorläuferzellen und/oder die Neurone während der Befeldung einer magnetischen Feldstärke von 0,05 bis 1,5 pT, bevorzugt 0,1 bis 0,5 pT, ferner bevorzugt von 0,125 pT ausgesetzt sind.

7. *In vitro* Verfahren nach einem der Ansprüche 1 bis 6, **dadurch gekennzeichnet, dass** die neuronalen Vorläuferzellen und/oder die Neurone ein bis vier Mal pro Woche, bevorzugt zwei Mal pro Woche befeldet werden.

8. *In vitro* Verfahren nach einem der Ansprüche 1 bis 7, **dadurch gekennzeichnet, dass** die neuronalen Vorläuferzellen und/oder die Neurone jeweils für mindestens 30 Minuten, bevorzugt für 30 bis 60 Minuten, ferner bevorzugt für 60 Minuten befeldet werden.

9. *In vitro* Verfahren nach einem der Ansprüche 1 bis 8, ferner umfassend den Schritt des Zugebens eines Antidementivas, bevorzugt eines Acetylcholinesterasehemmers, zu den Zellen.

10. *In vitro* Verfahren nach einem der Ansprüche 1 bis 9, **dadurch gekennzeichnet, dass** die neuronalen Vorläuferzellen und/oder die Neurone mindestens während der ersten 28 Tage in Kultur, bevorzugt mindestens während der ersten 10 Tage in Kultur, befeldet werden.

11. *In vitro* Verfahren nach einem der Ansprüche 1 bis 10, **dadurch gekennzeichnet, dass** die Neurone Interneurone sind.

## Claims

1. *In vitro* method for promoting the development of neurons, comprising the steps of
- culturing the neurons from neuronal precursor cells, and
- field treatment of the neuronal precursor cells and/or the neurons at a high-frequency oscillation having a frequency of from 100 to 200 MHz during culturing,
**characterized in that** the high-frequency oscillation is modulated by a low-frequency oscillation having a frequency of from 5 to 20 Hz.

2. *In vitro* method according to Claim 1, **characterized in that** the high-frequency oscillation has a frequency of from 130 to 170 MHz, more preferably 150 MHz.

3. *In vitro* method according to Claim 1 or 2, **characterized in that** the low-frequency oscillation has a frequency of from 10 to 20 Hz, preferably 10 Hz or 16 Hz.

4. *In vitro* method according to any of Claims 1 to 3, **characterized in that** the high-frequency oscillation is modulated in an alternating manner by a low-frequency oscillation having a frequency of 10 Hz and 16 Hz.

5. *In vitro* method according to any of Claims 1 to 4, **characterized in that** the neuronal precursor cells and/or the neurons are subjected to field treatment by means of an irradiation device and are arranged at a distance of from 1 up to 100 mm, preferably from 30 to 100 mm, more preferably from 50 to 100 mm, from the irradiation device.

6. *In vitro* method according to any of Claims 1 to 4, **characterized in that** the neuronal precursor cells and/or the neurons are exposed to a magnetic field strength of from 0.05 to 1.5 pT, preferably from 0.1 to 0.5 pT, more preferably 0.125 pT, during the field treatment.

7. *In vitro* method according to any of Claims 1 to 6, **characterized in that** the neuronal precursor cells and/or the neurons are subjected to field treatment from once per week to four times per week, preferably twice per week.

8. *In vitro* method according to any of Claims 1 to 7, **characterized in that** the neuronal precursor cells and/or the neurons are subjected to field treatment for at least 30 minutes, preferably for from 30 to 60 minutes, more preferably for 60 minutes, in each case.

9. *In vitro* method according to any of Claims 1 to 8, further comprising the step of adding an anti-dementia agent, preferably an acetylcholinesterase inhibitor, to the cells.

10. *In vitro* method according to any of Claims 1 to 9, **characterized in that** the neuronal precursor cells and/or the neurons are subjected to field treatment at least during the first 28 days in culture, preferably at least during the first 10 days in culture.

11. *In vitro* method according to any of Claims 1 to 10, **characterized in that** the neurons are interneurons.

## Revendications

1. Procédé *in vitro* pour favoriser le développement de neurones, comprenant les étapes suivantes :
- la culture des neurones à partir de cellules précurseurs neuronales, et
- l'exposition des cellules précurseurs neuronales et/ou des neurones à un champ pendant la culture avec une oscillation haute fréquence qui présente une fréquence allant de 100 à 200 MHz,
**caractérisé en ce que** l'oscillation haute fréquence est modulée par une oscillation basse fréquence qui présente une fréquence allant de 5 à 20 Hz.

2. Procédé *in vitro* selon la revendication 1, **caractérisé en ce que** l'oscillation haute fréquence présente une fréquence allant de 130 à 170 MHz, de manière davantage préférée de 150 MHz.

3. Procédé *in vitro* selon la revendication 1 ou 2, **caractérisé en ce que** l'oscillation basse fréquence présente une fréquence allant de 10 à 20 Hz, de préférence de 10 Hz ou de 16 Hz.

4. Procédé *in vitro* selon l'une quelconque des revendications 1 à 3, **caractérisé en ce que** l'oscillation haute fréquence est modulée en alternance par une oscillation basse fréquence ayant une fréquence de 10 Hz et de 16 Hz.

5. Procédé *in vitro* selon l'une quelconque des revendications 1 à 4, **caractérisé en ce que** les cellules précurseurs neuronales et/ou les neurones sont exposés à un champ par un dispositif d'exposition à un rayonnement, et sont agencés à un écart de 1 à 100 mm, de préférence de 30 à 100 mm, de manière davantage préférée de 50 à 100 mm, du dispositif d'exposition à un rayonnement.

6. Procédé *in vitro* selon l'une quelconque des revendications 1 à 4, **caractérisé en ce que** les cellules précurseurs neuronales et/ou les neurones sont exposés pendant l'exposition à un champ à une force de champ magnétique de 0,05 à 1,5 pT, de préférence de 0,1 à 0,5 pT, de manière davantage préférée de 0,125 pT.

7. Procédé *in vitro* selon l'une quelconque des revendications 1 à 6, **caractérisé en ce que** les cellules précurseurs neuronales et/ou les neurones sont exposés à un champ une à quatre fois par semaine, de préférence deux fois par semaine.

8. Procédé *in vitro* selon l'une quelconque des revendications 1 à 7, **caractérisé en ce que** les cellules précurseurs neuronales et/ou les neurones sont à chaque fois exposés à un champ pendant au moins 30 minutes, de préférence pendant 30 à 60 minutes, de manière davantage préférée pendant 60 minutes.

9. Procédé *in vitro* selon l'une quelconque des revendications 1 à 8, comprenant en outre l'étape d'ajout d'un médicament contre les démences, de préférence d'un inhibiteur d'acétylcholine-estérase, aux cellules.

10. Procédé *in vitro* selon l'une quelconque des revendications 1 à 9, **caractérisé en ce que** les cellules précurseurs neuronales et/ou les neurones sont exposés à un champ au moins pendant les 28 premiers jours en culture, de préférence au moins pendant les 10 premiers jours en culture.

11. Procédé *in vitro* selon l'une quelconque des revendications 1 à 10, **caractérisé en ce que** les neurones sont des interneurones.
